(19) Europäisches Patentamt
European Patent Office
Office européen des brevets

(11) EP 3 037 545 A1

(12) **EUROPEAN PATENT APPLICATION**

(43) Date of publication:
**29.06.2016 Bulletin 2016/26**

(51) Int Cl.:
***C12Q 1/68*** *(2006.01)*

(21) Application number: **14199999.5**

(22) Date of filing: **23.12.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **The Provost, Fellows, Foundation Scholars, & the other members of Board, of the College of the Holy & Undiv. Trinity of Queen Elizabeth near Dublin**
**Dublin 2 (IE)**

(72) Inventor: **Perry, Antoinette**
**Dublin, D8 (IE)**

(74) Representative: **Carmody, Mark et al**
**PurdyLucey**
**Intellectual Property**
**6-7 Harcourt Terrace**
**Dublin 2 (IE)**

(54) **A DNA-methylation test for prostate cancer**

(57) A method of determining the risk of metastatic prostate cancer in an individual diagnosed with prostate cancer, the method comprising a step of assaying a biological sample obtained from the individual for the presence of at last one methylated regulatory DNA sequence selected from group comprising: SEQUENCE ID No's 1 to 17, or variants thereof, and correlating the presence or absence of the methylated regulatory DNA sequence with aggressive (metastatic) prostate cancer.

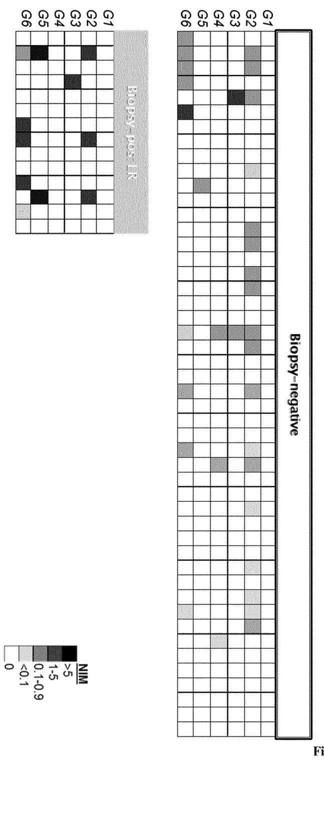


Figure 1



EP 3 037 545 A1

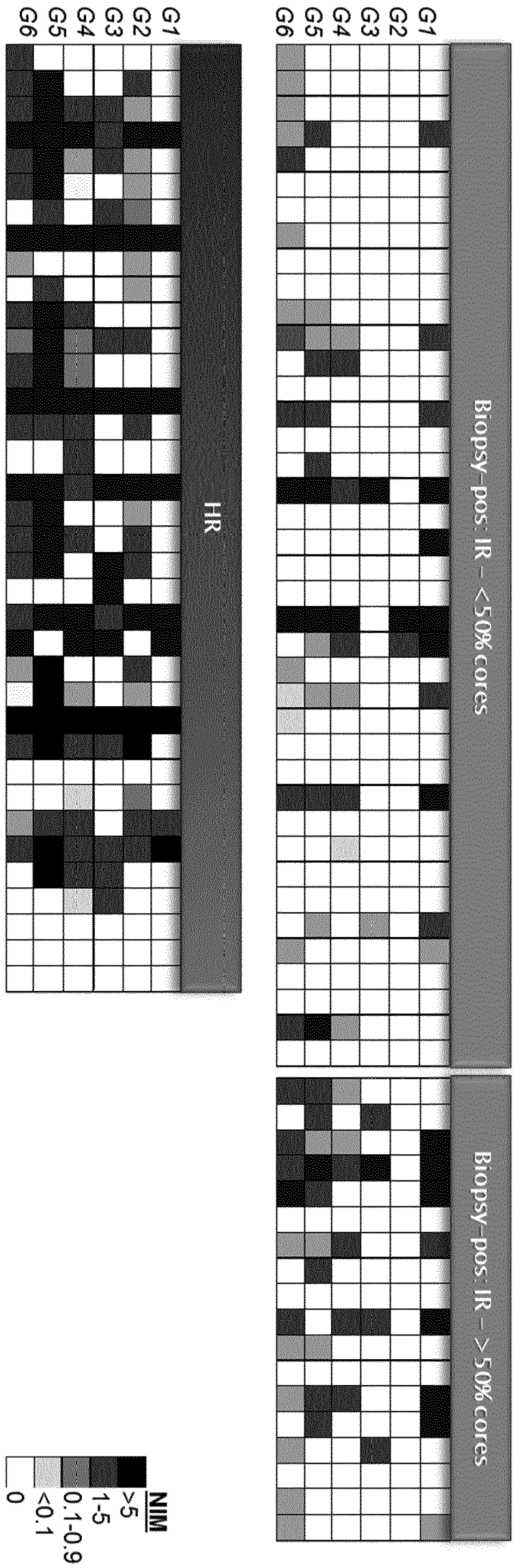


Figure 1 (continued)

SEQUENCE LISTING

<110> The Provost, Fellows, Foundation Scholars, & the other members of Board, of the College of the Holy & Unidv. Trinity of Queen Elizabeth, near Dublin

<120> A DNA-Methylation Test for Prostate Cancer

<130> P11494EP00

<160> 72

<170> PatentIn version 3.5

<210> 1
<211> 92
<212> DNA
<213> Homo sapiens

<400> 1
gctgcgcggc gactccgggg actccagggc gcccctctgc ggccgacgcc cggggtgcag 60

cggccgccgg ggctggggcc ggcgggagtc cg 92

<210> 2
<211> 129
<212> DNA
<213> Homo sapiens

<400> 2
agccccccgg agctgcgcgc gggcttgcag cgcctcgccc gcgctgtcct cccggtgtcc 60

cgcttctccg cgccccagcc gccggctgcc agcttttcgg ggccccgagt cgcacccagc 120

gaagagagc 129

<210> 3
<211> 78
<212> DNA
<213> Homo sapiens

<400> 3
tttccccgac accggctcgc cgcagggaga cctcaccccg agagcggaag gggtaagggc 60

ggcggggtca aggagatc 78

<210> 4
<211> 77
<212> DNA
<213> Homo sapiens

<400> 4
aagcgggcgt gagaccgagc gcccatgggc cggtcacgcc gggtgcccgc tcaccccccg 60

acgccagcag gagcgcg 77

<210> 5
<211> 75
<212> DNA

<213> Homo sapiens

<400> 5
cggaccaggg cgctccccat tcccgtcggg agcccgccga ttggctgggt gtgggcgcac 60

gtgaccgaca tgtgg 75

<210> 6
<211> 76
<212> DNA
<213> Homo sapiens

<400> 6
cggaagcgct cgggcaaaga ctgcgaagaa gaaaagacat ctggcggaaa cctgtgcgcc 60

tggggcggtg gaactc 76

<210> 7
<211> 90
<212> DNA
<213> Homo sapiens

<400> 7
tgggctactc tggcttaacg ggaccagtag cagagcgccg cccgtcctgc ttgctgctgg 60

gtccggttgc cgaggcggaa aagtcgcaag 90

<210> 8
<211> 88
<212> DNA
<213> Homo sapiens

<400> 8
cctgctcctg gcctctgcag gcgcctgggc tgctccacct gagagctgcg cagggcggtc 60

tggcggtggc tgctgcccac ataatccg 88

<210> 9
<211> 121
<212> DNA
<213> Homo sapiens

<400> 9
cgccgaggtg ctgtgacctt cgtctcgcca actctctagc cccagcttca tctgcaaggt 60

gggcgggctg cttgcaaacc ggtcggcgtg gggtggggtc gcaccctcgg ctgcagcggc 120

c 121

<210> 10
<211> 86
<212> DNA
<213> Homo sapiens

<400> 10
gggtttcgtc gtcgttttcg ttcggaggtt ggagtgttgt tcgtcgggtc gtgcgttcgt 60

tcggtagcgg cgtgtattag tattat 86

<210> 11
<211> 95
<212> DNA
<213> Homo sapiens

<400> 11
cgctgggacg cacctgcctg ttcagtcccg caggcagagg ggctgcgcct gggcctacgc 60

tcgcgatacc cagaatccac tcgcacctgc gaagg 95

<210> 12
<211> 132
<212> DNA
<213> Homo sapiens

<400> 12
ctgccggagg acgcgctgct gcgagaccag ccgcggcgtc tttggcagta gtgggcgtgc 60

ttgcgggtcc aggagggccc ctctcccgcg accgccgacc acgatgagag cgtgaagacc 120

ctctcgaaag ga 132

<210> 13
<211> 79
<212> DNA
<213> Homo sapiens

<400> 13
cggtcctggc tcaaactctc gtcacctgat cctcggcgaa agctcctatc gcgacacttt 60

gaccgtagcc gttcccgcc 79

<210> 14
<211> 135
<212> DNA
<213> Homo sapiens

<400> 14
acgtgtggcc tgtcacaccg tcggctgtat tggatcagga ttatttctta tgaaggtctg 60

ctttgccagt acgcagcagg tcccagtctc tacgccgtcc cgaacacctc gtagaaatac 120

ggggcatgca caacg 135

<210> 15
<211> 101
<212> DNA
<213> Homo sapiens

<400> 15
cggattccgt tcatgcctct cggagctaca gggcttggtg cggagacgca gggcgggcgc 60

gctgggctct gggtgtccgc aacccaaagt gcagctggtg t 101

<210> 16
<211> 100

<212> DNA
<213> Homo sapiens

<400> 16
cgggtcgcct ccacctcccg cagccgccgc gccccggctc gccgcgctcc cgccggcacc 60

atggaggaat tcctgggaca accgcctgca gccggcgact 100

<210> 17
<211> 98
<212> DNA
<213> Homo sapiens

<400> 17
ggtggaggca gccagggctt acctgtacac tgacttgaga ccagttgaat aaaagtgcac 60

accttaaaaa tgaggccaag tgtgactttg tggtgtgg 98

<210> 18
<211> 1335
<212> DNA
<213> Homo sapiens

<400> 18
agccccaagc ttaccacctg cacccggaga gctgtgtcac catgtgggtc ccggttgtct 60

tcctcaccct gtccgtgacg tggattggtg ctgcacccct catcctgtct cggattgtgg 120

gaggctggga gtgcgagaag cattcccaac cctggcaggt gcttgtggcc tctcgtggca 180

gggcagtctg cggcggtgtt ctggtgcacc cccagtgggt cctcacagct gcccactgca 240

tcaggaagcc aggtgatgac tccagccacg acctcatgct gctccgcctg tcagagcctg 300

ccgagctcac ggatgctgtg aaggtcatgg acctgcccac ccaggagcca gcactgggga 360

ccacctgcta cgcctcaggc tggggcagca ttgaaccaga ggagttcttg accccaaaga 420

aacttcagtg tgtggacctc catgttattt ccaatgacgt gtgtgcgcaa gttcaccctc 480

agaaggtgac caagttcatg ctgtgtgctg gacgctggac agggggcaaa agcacctgct 540

cgggtgattc tgggggccca cttgtctgta atggtgtgct tcaaggtatc acgtcatggg 600

gcagtgaacc atgtgccctg cccgaaaggc cttccctgta caccaaggtg gtgcattacc 660

ggaagtggat caaggacacc atcgtggcca acccctgagc acccctatca accccctatt 720

gtagtaaact tggaaccttg gaaatgacca ggccaagact caagcctccc cagttctact 780

gacctttgtc cttaggtgtg aggtccaggg ttgctaggaa aagaaatcag cagacacagg 840

tgtagaccag agtgtttctt aaatggtgta attttgtcct ctctgtgtcc tggggaatac 900

tggccatgcc tggagacata tcactcaatt tctctgagga cacagatagg atggggtgtc 960

tgtgttattt gtggggtaca gagatgaaag aggggtggga tccacactga gagagtggag 1020

agtgacatgt gctggacact gtccatgaag cactgagcag aagctggagg cacaacgcac 1080

cagacactca cagcaaggat ggagctgaaa acataaccca ctctgtcctg gaggcactgg 1140

gaagcctaga gaaggctgtg agccaaggag ggagggtctt cctttggcat gggatgggga 1200

tgaagtaagg agagggactg gaccccctgg aagctgattc actatggggg gaggtgtatt 1260

gaagtcctcc agacaacccct cagatttgat gatttcctag tagaactcac agaaataaag 1320

agctgttata ctgtg 1335

<210> 19
<211> 648
<212> DNA
<213> Artificial Sequence

<220>
<223> Synthetic Block Sequence

<400> 19
aaaggtggag gtagttaggg tttatttgta tattgatttg agattagttg aataaaagtg 60

tatattttaa aaatgaggtt aagtgtgatt ttgtggtgtg gaaagttgcg cggcgatttc 120

ggggatttta gggcgttttt ttgcggtcga cgttcggggt gtagcggtcg tcggggttgg 180

ggtcggcggg agttcgaaaa gtttttcgga gttgcgcgcg ggtttgtagc gtttcgttcg 240

cgttgttttt tcggtgtttc gtttttttcgc gttttagtcg tcggttgtta gtttttcggg 300

gtttcgagtc gtatttagcg aagagagcaa attttttcga tatcggttcg tcgtagggag 360

attttatttc gagagcggaa ggggtaaggg cggcggggtt aaggagatca aaaagcgggc 420

gtgagatcga gcgtttatgg gtcggttacg tcgggtgttc gtttattttt cgacgttagt 480

aggagcgcga aattatatgt cggttacgtg cgtttatatt tagttaatcg gcgggttttc 540

gacgggaatg gggagcgttt tggttcaaac ggaagcgttc gggtaaagat tgcgaagaag 600

aaaagatatt tggcggaaat ttgtgcgttt ggggcggtgg aattcaaa 648

<210> 20
<211> 885
<212> DNA
<213> Artificial Sequence

<220>
<223> Synthetic Block Sequence

<400> 20
aaaggtggag gtagttaggg tttatttgta tattgatttg agattagttg aataaaagtg 60

tatattttaa aaatgaggtt aagtgtgatt ttgtggtgtg gaaatgggtt attttggttt 120

aacgggatta gtagtagagc gtcgttcgtt ttgtttgttg ttgggttcgg ttgtcgaggc 180

ggaaaagtcg taagaaattt gtttttggtt tttgtaggcg tttgggttgt tttatttgag 240

agttgcgtag ggcggtttgg cggtggttgt tgtttatata attcgaaacg tcgaggtgtt 300

gtgattttcg tttcgttaat tttttagttt tagttttatt tgtaaggtgg gcgggttgtt 360

tgtaaatcgg tcggcgtggg gtggggtcgt attttcggtt gtagcggtta aagggtttcg 420

tcgtcgtttt cgttcggagg ttggagtgtt gttcgtcggg tcgtgcgttc gttcggtagc 480

ggcgtgtatt agtattataa acgttgggac gtatttgttt gtttagtttc gtaggtagag 540

gggttgcgtt tgggtttacg ttcgcgatat ttagaattta ttcgtatttg cgaaggcgaa 600

aatacgtttt tgtcggtcgt ttagtttttt gtaggtgtaa gggcggatgt tttagcgatt 660

acgggagtcg ggttggggag gttggtgggg ggcgggggga gttttaaatt taggttcgtt 720

tagttatagg cgtttaggtt tagtcggaaa ttgtcggagg acgcgttgtt gcgagattag 780

tcgcggcgtt tttggtagta gtgggcgtgt ttgcgggttt aggagggttt ttttttcgcg 840

atcgtcgatt acgatgagag cgtgaagatt ttttcgaaag gaaaa 885

<210> 21
<211> 643
<212> DNA
<213> Artificial Sequence

<220>
<223> Synthetic Block Sequence

<400> 21
aaagggcggt tttggtttaa attttcgtta tttgattttc ggcgaaagtt tttatcgcga 60

tattttgatc gtagtcgttt tcgttaaaaa aggtggaggt agttagggtt tatttgtata 120

ttgatttgag attagttgaa taaaagtgta tattttaaaa atgaggttaa gtgtgatttt 180

gtggtgtgga aattcggttt attacggcgg ttatttttcg ggtagtgacg acgatcgaga 240

cggtgagggc ggttatcgtt ggggagggag gttcgggttt aggtttggaa gtaaaacgtg 300

tggtttgtta tatcgtcggt tgtattggat taggattatt ttttatgaag gtttgttttg 360

ttagtacgta gtaggtttta gtttttacgt cgtttcgaat atttcgtaga aatacggggt 420

atgtataacg aaacggattt cgtttatgtt tttcggagtt atagggtttg gtgcggagac 480

gtagggcggg cgcgttgggt tttgggtgtt cgtaatttaa agtgtagttg gtgtaaacgg 540

gtcgttttta tttttcgtag tcgtcgcgtt tcggttcgtc gcgttttcgt cggtattatg 600

gaggaatttt tgggataatc gtttgtagtc ggcgattggg aaa 643

<210> 22
<211> 27
<212> DNA
<213> Artificial Sequence

<220>
<223> Primer ACTB

<400> 22
ggtggaggta gttagggttt atttgta 27

<210> 23
<211> 30
<212> DNA
<213> Artificial Sequence

<220>
<223> Primer ACTB

<400> 23
ccacaccaca aaatcacact taacctcatt 30

<210> 24
<211> 17
<212> DNA
<213> Artificial Sequence

<220>
<223> Primer GSTP1

<400> 24
gttgcgtggc gatttcg 17

<210> 25
<211> 16
<212> DNA
<213> Artificial Sequence

<220>
<223> Primer GSTP1

<400> 25
cgaactcccg ccgacc 16

<210> 26
<211> 19
<212> DNA
<213> Artificial Sequence

<220>
<223> Primer SFRP2

<400> 26
agtttttcgg agttgcgcg 19

<210> 27
<211> 24
<212> DNA
<213> Artificial Sequence

<220>
<223> Primer SFRP2

<400> 27
gctctcttcg ctaaatacga ctcg 24

<210> 28
<211> 22
<212> DNA

<213> Artificial Sequence

<220>
<223> Primer IGFBP3

<400> 28
tttttcgat atcggttcgt cg 22

<210> 29
<211> 19
<212> DNA
<213> Artificial Sequence

<220>
<223> Primer IGFBP3

<400> 29
gatctcctta accccgccg 19

<210> 30
<211> 17
<212> DNA
<213> Artificial Sequence

<220>
<223> Primer IGFBP7

<400> 30
aagcgggcgt gagatcg 17

<210> 31
<211> 19
<212> DNA
<213> Artificial Sequence

<220>
<223> Primer IGFBP7

<400> 31
cgcgctccta ctaacgtcg 19

<210> 32
<211> 27
<212> DNA
<213> Artificial Sequence

<220>
<223> Primer APC

<400> 32
ttatatgtcc gttacgtgcg tttatat 27

<210> 33
<211> 19
<212> DNA
<213> Artificial Sequence

<220>

<223> Primer APC

<400> 33
gaaccaaaac gctccccat 19

<210> 34
<211> 19
<212> DNA
<213> Artificial Sequence

<220>
<223> Primer PTGS2

<400> 34
cggaagcgtt cgggtaaag 19

<210> 35
<211> 20
<212> DNA
<213> Artificial Sequence

<220>
<223> Primer PTGS2

<400> 35
gaattccacc gccccaaacg 20

<210> 36
<211> 22
<212> DNA
<213> Artificial Sequence

<220>
<223> Primer LXN

<400> 36
tgggttattt tggtttaacg gg 22

<210> 37
<211> 20
<212> DNA
<213> Artificial Sequence

<220>
<223> Primer LXN

<400> 37
cttacgactt ttccgcctcg 20

<210> 38
<211> 23
<212> DNA
<213> Artificial Sequence

<220>
<223> Primer MAGPIE-1

<400> 38

tttgtttttg gtttttgtag gcg 23

<210> 39
<211> 25
<212> DNA
<213> Artificial Sequence

<220>
<223> Primer MAGPIE-1

<400> 39
cgaattatat aaacaacaac caccg 25

<210> 40
<211> 20
<212> DNA
<213> Artificial Sequence

<220>
<223> Primer DNAH10

<400> 40
cgtcgaggtg ttgtgatttt 20

<210> 41
<211> 22
<212> DNA
<213> Artificial Sequence

<220>
<223> Primer DNAH10

<400> 41
aaccgctaca accgaaaata cg 22

<210> 42
<211> 17
<212> DNA
<213> Artificial Sequence

<220>
<223> Primer ZMIZ1

<400> 42
gggtttcgtc gtcgttt 17

<210> 43
<211> 22
<212> DNA
<213> Artificial Sequence

<220>
<223> Primer ZMIZ1

<400> 43
ataatactaa tacacgccgc ta 22

<210> 44
<211> 21
<212> DNA
<213> Artificial Sequence

<220>
<223> Primer CENPV

<400> 44
cgttgggacg tatttgtttg t 21

<210> 45
<211> 20
<212> DNA
<213> Artificial Sequence

<220>
<223> Primer CENPV

<400> 45
cgccttcgca aatacgaata 20

<210> 46
<211> 17
<212> DNA
<213> Artificial Sequence

<220>
<223> Primer OR2L13

<400> 46
ttgtcggagg acgcgtt 17

<210> 47
<211> 23
<212> DNA
<213> Artificial Sequence

<220>
<223> Primer OR2L13

<400> 47
tcctttcgaa aaaatcttca cgc 23

<210> 48
<211> 24
<212> DNA
<213> Artificial Sequence

<220>
<223> Primer MTMR8

<400> 48
cgggttttta ttttcgtttt ttcg 24

<210> 49
<211> 24
<212> DNA

<213> Artificial Sequence

<220>
<223> Primer MTMR8

<400> 49
aaaataacgt agtcgttttc gatc 24

<210> 50
<211> 21
<212> DNA
<213> Artificial Sequence

<220>
<223> Primer F3

<400> 50
tgtggtttgt tatatcgtcg g 21

<210> 51
<211> 23
<212> DNA
<213> Artificial Sequence

<220>
<223> Primer F3

<400> 51
aatacaacgt tatacatacc ccg 23

<210> 52
<211> 23
<212> DNA
<213> Artificial Sequence

<220>
<223> Primer CDH8

<400> 52
cggatttcgt ttatgttttt cgg 23

<210> 53
<211> 24
<212> DNA
<213> Artificial Sequence

<220>
<223> Primer CDH8

<400> 53
acaccaacta cactttaaat tacg 24

<210> 54
<211> 23
<212> DNA
<213> Artificial Sequence

<220>

<223> Primer GALNTL6

<400> 54
cgggtcgttt ttatttttcg tag 23

<210> 55
<211> 18
<212> DNA
<213> Artificial Sequence

<220>
<223> Primer GALNTL6

<400> 55
aatcgccgac tacaaacg 18

<210> 56
<211> 21
<212> DNA
<213> Artificial Sequence

<220>
<223> Probe ACTB

<400> 56
cacttttatt caactaatct c 21

<210> 57
<211> 15
<212> DNA
<213> Artificial Sequence

<220>
<223> Probe GSTP1

<400> 57
cgacgaccgc tacac 15

<210> 58
<211> 17
<212> DNA
<213> Artificial Sequence

<220>
<223> Probe SFRP2

<400> 58
tgtagcgttt cgttcgc 17

<210> 59
<211> 21
<212> DNA
<213> Artificial Sequence

<220>
<223> Probe IFGBP3

<400> 59

agattttatt tcgagagcgg a 21

<210> 60
<211> 19
<212> DNA
<213> Artificial Sequence

<220>
<223> Probe IGFBP7

<400> 60
ttatgggtcg gttacgtcg 19

<210> 61
<211> 22
<212> DNA
<213> Artificial Sequence

<220>
<223> Probe APC

<400> 61
cccgtcgaaa acccgccgat ta 22

<210> 62
<211> 29
<212> DNA
<213> Artificial Sequence

<220>
<223> Probe PTGS2

<400> 62
tttccgccaa atatcttttc ttcttcgca 29

<210> 63
<211> 27
<212> DNA
<213> Artificial Sequence

<220>
<223> Probe LXN

<400> 63
cgtcgttcgt tttgtttgtt gttgggt 27

<210> 64
<211> 22
<212> DNA
<213> Artificial Sequence

<220>
<223> Probe MAGPIE-1

<400> 64
tttgagagtt gcgtagggcg gt 22

<210> 65
<211> 25
<212> DNA
<213> Artificial Sequence

<220>
<223> Probe DNAH10

<400> 65
cgggttgttt gtaaatcggt cggcg 25

<210> 66
<211> 24
<212> DNA
<213> Artificial Sequence

<220>
<223> Probe ZMIZ1

<400> 66
ttcggaggtt ggagtgttgt tcgt 24

<210> 67
<211> 24
<212> DNA
<213> Artificial Sequence

<220>
<223> Probe CENPV

<400> 67
ttgcgtttgg gtttacgttc gcga 24

<210> 68
<211> 25
<212> DNA
<213> Artificial Sequence

<220>
<223> Probe OR2L13

<400> 68
tgcgagatta gtcgcggcgt ttttg 25

<210> 69
<211> 27
<212> DNA
<213> Artificial Sequence

<220>
<223> Probe MTMR8

<400> 69
tgattttcgg cgaaagtttt tatcgcg 27

<210> 70
<211> 31
<212> DNA

<213> Artificial Sequence

<220>
<223> Probe F3

<400> 70
cgtagtaggt tttagttttt acgtcgtttc g 31

<210> 71
<211> 17
<212> DNA
<213> Artificial Sequence

<220>
<223> Probe CDH8

<400> 71
cgtagggcgg gcgcgtt 17

<210> 72
<211> 19
<212> DNA
<213> Artificial Sequence

<220>
<223> Probe GALNTL6

<400> 72
cgcgtttcgg ttcgtcgcg 19

**Description**

## Technical Field

The invention relates to the detection of a biomarker in a biological sample to test for the presence of prostate cancer. Specifically, the invention relates to the detection of a plurality of biomarkers in a biological sample to distinguish the presence of aggressive prostate cancer from non-aggressive prostate cancer or no cancer.

## Background to the Invention

Prostate cancer (PCa) is the most common non-cutaneous malignancy in men in the Western world. An estimated 1.1 million new cases were diagnosed in 2012, accounting for 15% of all male cancers worldwide. Ireland is currently experiencing one of the highest incidences of PCa in Europe, with approximately 3,000 new cases diagnosed per annum, representing 30% of all invasive cancers in men. With an ageing Western population and spread of Western culture (particularly diet), the global incidence is predicted to rise dramatically; the National Cancer Registry predicts the incidence in Ireland to rise by between 104-288% by 2040.

It is often said that "most men die *with* and *not because of* their prostate cancer". This is explained by the fact that most prostate tumours have a slow, long natural trajectory, posing little likelihood of clinical manifestation, and deemed indolent in nature; 10-year survival rates for PCa are close to 100%. Nevertheless, a proportion of prostate tumours are highly aggressive, and are associated with the lethal form of the disease. Whilst PSA (prostate specific antigen) screening and improvements in treatments have reduced PCa mortality, this disease accounted for an ~307,000 deaths in 2012, making it the $5^{th}$ leading cause of male cancer-related deaths worldwide. Identifying molecular correlates to discern between aggressive and indolent tumors at an early stage (whilst potentially curable), is one of the greatest unmet clinical needs in this field. This will become even more pressing as the differential between the total number of PCa cases diagnosed and the number of lethal PCa cases grows.

Early detection and diagnosis of PCa involves a combination of a PSA blood test, a digital rectal examination (DRE) and histological examination of transrectal ultrasound (TRUS)-guided biopsy cores, respectively. Several major problems confound the early detection of PCa. There are an estimated 25-45 million PSA tests performed worldwide every year, Widespread PSA testing has significantly increased PCa incidence and led to overtreatment of low-risk disease with little likelihood of clinical manifestation. A further problem with PSA is its poor tumour-specificity; its high false-positive rate means that two-thirds of men who undergo invasive TRUS-biopsy have no tumour diagnosed. There are an estimated 10 million prostate biopsies performed worldwide/annum. Unnecessary TRUS-biopsies create an enormous burden on our healthcare system and cause significant anxiety, trauma and co-morbidities for patients. Finally, TRUS-biopsies are needle biopsies that sample <5% of the prostate and can thus miss tumour foci or indeed miss high-grade aggressive tumours. Studies addressing the economic burden of cancer in the EU, have estimated costs for PCa diagnosis and treatment over the next 20 years per 100,000 men at €30,284,000 (unscreened population) and €60,695,000 (screened population), €23,669,000 of which can be attributed to over-detected cancers.

Currently, there are no commercially available molecular diagnostics for PCa in widespread clinical practice. Progensa® (Gen-Probe) is a urine-based test of *PCA3* gene expression performed after DRE, with FDA approval for use in men who have had ≥1 previous negative biopsies and for whom a repeat biopsy would be recommended based on current standard of care. The test is used to guide the decision to perform a repeat biopsy only. Its prognostic value is debated and research efforts combining it with the fusion-transcript *TMPRSS2-ERG* are underway in an attempt to address this.

Prolaris® (Myriad Genetics) and onco*type*DX® Prostate Cancer Assay (Genomic Health) are two examples of prognostic gene expression signatures (46 genes and 17 genes, respectively) that are analysed on biopsy tissues to aid prediction of PCa aggressiveness in conjunction with other clinical parameters (Gleason score, PSA). Both tests provide a more individualised risk-assessment of the underlying biology of the patient's tumour and are therefore aimed at guiding the decision between active surveillance and radical treatment in men diagnosed with PCa.

MDxHealth's product ConfirmMDx™ is a PCR-based assay, which measures methylation of a 3-gene panel (*GSTP1, RARβ, APC*) in biopsy cores. It is positioned to distinguish patients with a true-negative prostate biopsy from those with occult cancer and akin to Progensa®, is used to guide the decision to perform a repeat biopsy. This same 3-gene panel (ProCaM™) has also been investigated as a urine test to predict biopsy results for PCa, although these studies were inadequately powered.

It is an object of the subject invention to overcome at least one of the above-mentioned problems.

## Statements of Invention

In contrast to these prior art technologies, the test presented herein (called epigenetic Cancer of the Prostate test in urine or epiCaPture) is an example of a "first in field" for urine diagnostics of potentially lethal, high-risk PCa. The panel of genes encompasses multiple dysregulated pathways in PCa, which is necessary to address the heterogeneity of the disease. These pathways include intracellular detoxification, the IGF axis, the Wnt axis and inflammation. The test presented herein addresses the unmet clinical needs confounding early detection of PCa. The test is a non-invasive DNA methylation test performed using urine or urine cell-sediment. It comprises a panel of at least 6 genes and an internal control gene. The test described herein offers significant commercial potential as a liquid biopsy for early non-invasive detection of high-risk, potentially lethal PCa. The data show that the test offers the unique advantages of i) better tumour-specificity than PSA, and ii) selective identification of high-risk PCa.

According to the invention, there is provided, as set out in the appended claims, a method of determining the risk of aggressive prostate cancer in an individual diagnosed with prostate cancer, comprising a step of assaying a biological sample obtained from the individual for the presence of at least one methylated regulatory DNA sequence selected from group comprising SEQ ID NOs. to 1 to 16, or variants thereof, and correlating the presence of the at least one methylated regulatory DNA sequence with the risk of aggressive prostate cancer. The methylated regulatory DNA sequences as defined by SEQ ID NOs. 1 to 16 correspond to the genes *GSTP1, SFRP2, IGFBP3, IGFBP7, APC, PTGS-2, LXN, MAGPIE-1, DNAH10, ZMIZ1, CENPV, OR2L13, MTMR8, F3, CDH8* and *GALNTL6,* respectively, and correlating the presence of the methylated regulatory DNA sequence(s) of these genes with an increased risk of aggressive prostate cancer. The presence of the methylated regulatory DNA sequence may be detected directly by quantitatively assaying for methylation of the DNA regulatory sequences located adjacent to the associated gene(s).

In one embodiment, the method further comprises the step of assaying for the presence of the *PSA* gene in addition to at least one methylated regulatory DNA sequence corresponding to the genes selected from group comprising *GSTP1, SFRP2, IGFBP3, IGFBP7, APC, PTGS-2, LXN, MAGPIE-1, DNAH10, ZMIZ1, CENPV, OR2L13, MTMR8, F3, CDH8* and *GALNTL6,* wherein the presence of PSA in combination with positive detection of the at least one methylated regulatory DNA sequence corresponding to the genes selected from group comprising *GSTP1, SFRP2, IGFBP3, IGFBP7, APC, PTGS-2, LXN, MAGPIE-1, DNAH10, ZMIZ1, CENPV, OR2L13, MTMR8, F3, CDH8* and *GALNTL6,* correlates with an increased risk of aggressive prostate cancer compared with an individual with prostate cancer who is positive for PSA detection but negative for detection of the at least one methylated regulatory DNA sequence.

In a preferred embodiment of the invention, the method involves assaying for methylation of a DNA regulatory sequence specific to the or each gene selected from group comprising *GSTP1, SFRP2, IGFBP3, IGFBP7, APC, PTGS-2, LXN, MAGPIE-1, DNAH10, ZMIZ1, CENPV, OR2L13, MTMR8, F3, CDH8* and *GALNTL6.* Preferably, the DNA regulatory sequence is one or both of SEQ ID NOs 1 or 3, or variants thereof, which are specific to the biomarkers *GSTP1* and *IGFBP3,* respectively. Specifically, the DNA regulatory sequence is one or more selected from SEQ ID NOs: 1 to 16, or variants thereof, and is specific to the biomarkers selected from *GSTP1, SFRP2, IGFBP3, IGFBP7, APC, PTGS-2, LXN, MAGPIE-1, DNAH10, ZMIZ1, CENPV, OR2L13, MTMR8, F3, CDH8* and *GALNTL6,* respectively. Preferably, the DNA regulatory sequence assayed for is all of SEQ ID NOs: 1 to 16, or variants thereof, and are specific to the biomarkers *GSTP1, SFRP2, IGFBP3, IGFBP7, APC, PTGS-2, LXN, MAGPIE-1, DNAH10, ZMIZ1, CENPV, OR2L13, MTMR8, F3, CDH8* and *GALNTL6,* respectively. Ideally, the DNA regulatory sequence assayed for is all of SEQ ID NOs: 1 to 6, or variants thereof, and are specific to the biomarkers *GSTP1, SFRP2, IGFBP3, IGFBP7, APC,* and *PTGS-2,* respectively. Preferably, assaying for methylation of the DNA regulatory sequence specific to the or each biomarker is combined with assaying for PSA (internal control, SEQ ID NO. 18).

In one embodiment, the method comprises assaying for at least four DNA regulatory sequence specific to their corresponding genes, at least three DNA regulatory sequences selected from the group comprising: SEQ ID NO.'s 1 to 16, or variants thereof, and at least one selected from the group comprising: SEQ ID NO.'s 17 and 18. Preferably, both SEQ ID NO. 17 and SEQ ID NO. 18 are determined in the method. In this way, the method of the invention employs at least three "positive" regulatory DNA sequences (*i.e.* a regulatory DNA sequence associated with presence of the cancer), and at least one "control" biomarker (*i.e.* a biomarker associated with prostate-derived DNA). Ideally, the "positive" regulatory DNA sequences are selected from the group comprising SEQUENCE ID NO's 1 to 6.

The two controls may be used in the method of the invention:

1) ACTB (SEQ ID NO. 17): ACTB is measured by quantitative PCR (qPCR) and verifies and quantifies the presence of bisulfite modified DNA in each test sample. The quantity of ACTB is used to calculate an epiCaPture score (a score derived from the method of the invention). The amount of each gene in the method must be normalised relative to the amount of input bisulfite modified DNA in each test sample.

2) KLK3 (SEQ ID NO. 18). Expression of the KLK3 gene (the gene encoding PSA, Prostate Specific Antigen) is measured by quantitative RT-PCR and is used as a positive control to confirm the presence of prostate-derived nucleic acids in the test sample. This is important to carry out, in order to show that a test sample which appears negative for prostate cancer as determined by the method of the invention, is indeed truly negative and it is not simply a virtue of no prostate-derived material present in the bio-specimen. The expression of the KLK3 gene is measured using a commercially available qPCR assay, such as Integrated DNA Technologies (Assay ID Hs.PT.58.38546086).

In one embodiment, the method comprises a step of assaying a biological sample obtained from the individual for the presence of a methylated regulatory DNA sequence from at least three sequences selected from group comprising: SEQUENCE ID NO's 1 to 16, or variants thereof, where the DNA regulatory sequences are specific to the biomarkers (genes) *GSTP1, SFRP2, IGFBP3, IGFBP7, APC, PTGS-2, LXN, MAGPIE-1, DNAH10, ZMIZ1, CENPV, OR2L13, MTMR8, F3, CDH8* and *GALNTL6.* In a preferred embodiment, the method comprises a step of assaying a biological sample for three, four, five, or six regulatory DNA sequence selected from group comprising: SEQUENCE ID NO's 1 to 16, or a variant thereof, where the regulatory DNA sequences are specific to the biomarkers (genes) *GSTP1, SFRP2, IGFBP3, IGFBP7, APC, PTGS-2, LXN, MAGPIE-1, DNAH10, ZMIZ1, CENPV, OR2L13, MTMR8, F3, CDH8* and *GALNTL6.* In a particularly preferred embodiment of the invention, the method comprises a step of assaying a biological sample obtained from the individual for the presence of a methylated regulatory DNA sequences defined by the group comprising, or consisting essentially of, SEQUENCE ID NO's 1 to 6, or variants thereof, where the regulatory DNA sequences are specific to the biomarkers (genes) *GSTP1, SFRP2, IGFBP3, IGFBP7, APC* and *PTGS-2.* Preferably, assaying for methylation of the regulatory DNA sequences, or variants thereof, where the regulatory DNA sequences are specific to the biomarkers (genes) *GSTP1, SFRP2, IGFBP3, IGFBP7, APC, PTGS-2,* is combined with assaying for PSA.

Where a patient is found to be positive for the methylated regulatory DNA sequences of SEQ ID NOs 1 to 6, or variants thereof, this correlates with a positive identification of prostate cancer at 97.92% specificity and a false positive rate of 0.04 versus 21% specificity and a false positive rate of 0.79 using PSA detection (applying a threshold of 4ng/ml).

Where a patient is found to be positive for all six of the methylated regulatory DNA sequences defined by SEQ ID NOs. 1 to 6, or variants thereof, this correlates with a positive identification of aggressive (high-risk) prostate cancer with a 79% sensitivity and an 82% specificity. The combination of the assay result of only two of these methylated regulatory DNA sequences, namely SEQ ID NO. 1 and SEQ ID NO. 3 (corresponding to the genes GSTP1 and IGFBP3, respectively) in this present analysis correlated with a positive detection of aggressive (high-risk or metastatic) prostate cancer with a sensitivity score of 82% and a specificity score of 82%.

In a preferred embodiment of the invention, the method comprises a step of assaying a biological sample from the individual for the presence of methylated regulatory DNA sequences defined by SEQ ID NO. 1 (GSTP1), and SEQ ID NO. 3 (IGFBP3), or variants thereof, optionally in combination with one or more methylated regulatory DNA sequences of genes selected from the group comprising: SEQ ID NO 2 (SFRP2), SEQ ID NO. 4 (IGFBP7), SEQ ID NO. 5 (APC), and SEQ ID NO. 6 (PTGS2), or variants thereof. The method may also comprise assaying a biological sample from the individual for the presence of methylated regulatory DNA sequences defined by SEQ ID NO. 1 (GSTP1), and SEQ ID NO. 3 (IGFBP3), or variants thereof, optionally in combination with one or more methylated regulatory DNA sequences of genes selected from the group comprising: SEQ ID NO 2 (SFRP2), SEQ ID NO. 4 (IGFBP7), SEQ ID NO. 5 (APC), and SEQ ID NO. 6 (PTGS2), SEQ ID NO. 7 (LXN), SEQ ID NO. 8 (MAGPIE-1), SEQ ID NO. 9 (DNAH10), SEQ ID NO. 10 (ZMIZ1), SEQ ID NO. 11 (CENPV), and SEQ ID NO. 12 (OR2L13), or variants thereof. The method may also comprise assaying a biological sample from the individual for the presence of methylated regulatory DNA sequences defined by SEQ ID NO. 1 (GSTP1), and SEQ ID NO. 3 (IGFBP3), or variants thereof, optionally in combination with one or more methylated regulatory DNA sequences of genes selected from the group comprising: SEQ ID NO 2 (SFRP2), SEQ ID NO. 4 (IGFBP7), SEQ ID NO. 5 (APC), and SEQ ID NO. 6 (PTGS2), SEQ ID NO. 7 (LXN), SEQ ID NO. 8 (MAGPIE-1), SEQ ID NO. 9 (DNAH10), SEQ ID NO. 10 (ZMIZ1), SEQ ID NO. 11 (CENPV), and SEQ ID NO. 12 (OR2L13), SEQ ID NO. 13 (MTMR8), SEQ ID NO. 14 (F3), SEQ ID NO. 15 (CDH8), and SEQ ID NO. 16 (GALNTL6), or variants thereof.

Preferably, the biological sample is assayed for the presence of PSA in combination with the methylated regulatory DNA sequences of any one or all of SEQUENCE ID NO's 1 to 6. Preferably, the biological sample is assayed for the presence of PSA in combination with the methylated regulatory DNA sequences of any one or all of SEQUENCE ID NO's 7 to 12. Preferably, the biological sample is assayed for the presence of PSA in combination with the methylated regulatory DNA sequences of any one or all of SEQUENCE ID NO's 13 to 16.

The invention also relates to a kit for assessing prostate cancer status in an individual, comprising components for detecting and/or measuring the level of a methylated regulatory DNA sequence of at least three selected from the group comprising: SEQUENCE ID No's 1 to 6.

The kit preferably comprises a pair of forward and reverse oligonucleotide primers (SEQ ID NOs. 22 to 55) designed to specifically hybridise with bisulfite modified hypermethylated DNA sequences at the regulatory regions of each specific gene as defined by SEQ ID NOs 1 to 16; a fluorescently labelled oligonucleotide probe designed to specifically hybridise with bisulfite modified hypermethylated DNA sequences at the regulatory region of each specific gene (SEQ ID NO. 56 to 72), a set of forward and reverse oligonucleotide primers and a fluorescently labelled probe to specifically hybridise with bisulfite modified DNA contained as part of the human ACTB gene, regardless of DNA methylation patterns of this gene (Positive control 1), a qRT-PCR assay for the KLK3 gene (Positive control 2) to control for the presence of prostate-derived nucleic acids in the bio-specimen, and a gBlock® synthetic gene fragments for construction of standard curves (SEQ ID NO. 19, 20 or 21), necessary for quantification of methylation levels at individual DNA sequences contained within the panel.

As indicated above, the methods, assays and kits of the invention employ biomarkers (methylated regulatory DNA sequences of specific genes or oligonucleotides specific to those regulatory DNA sequences of those genes) as a means of assessing the risk of an aggressive or metastatic prostate cancer in an individual. In one preferred embodiment of the invention, the methods, assays, and kits may be employed as a clinical screening tool to assist in the identification of individuals with an aggressive form of or a high risk metastatic prostate cancer, especially symptomatic individuals, who should be subjected to more invasive investigations, such as a prostate biopsy. In this regard, it should be noted that many patients who present with symptoms of prostate cancer (*i.e.* the need to urinate frequently, difficulty in starting urination, weak or interrupted flow of urine, painful/burning urination; blood in the urine *etc*.) can turn out to be negative for prostate cancer, yet still have to undergo a prostate biopsy to reach that diagnosis. In this regard, the present invention provides a useful clinical decision making tool which can assist a clinician in identifying those symptomatic patients that are most at risk of having the cancer, thereby potentially reducing the numbers of patients who have to undergo a prostate biopsy needlessly.

Thus, in one embodiment, the invention relates to a method of determining prostate cancer status in an individual, the method comprising a step of assaying a biological sample from the individual for a combination of methylated DNA regulatory sequences selected from SEQUENCE ID NO's: 1 to 16, which are the DNA regulatory sequences *GSTP1, SFRP2, IGFBP3, IGFBP7, APC, PTGS-2, LXN, MAGPIE-1, DNAH10, ZMIZ1, CENPV, OR2L13, MTMR8, F3, CDH8* and *GALNTL6,* respectively, the combination of methylated DNA regulatory sequences being chosen such that detection of at least one or more of the methylated DNA regulatory sequences in the individual correlates to at least a 50% risk of the individual being positive for aggressive or high risk metastatic prostate cancer. Typically, the combination of methylated DNA regulatory sequences is chosen such that detection of at least one or more of the methylated DNA regulatory sequences in the individual correlates to at least a 60% risk of the individual being positive for aggressive or high risk metastatic prostate cancer. Suitably, the combination of methylated DNA regulatory sequences is chosen such that detection of one or more of the methylated DNA regulatory sequences in the individual correlates to at least a 70% risk of the individual being positive for aggressive or high risk metastatic prostate cancer. Ideally, the combination of methylated DNA regulatory sequences is chosen such that detection of one or more of the methylated DNA regulatory sequences in the individual correlates to at least an 80% risk of the individual being positive for aggressive or high risk metastatic prostate cancer. Preferably, the detection of one or more methylated DNA regulatory sequences are combined with PSA.

Typically, the combination will comprise at least one methylated DNA regulatory sequences, at least two methylated DNA regulatory sequences, at least three methylated DNA regulatory sequences, preferably at least four methylated DNA regulatory sequences, more preferable would be at least five methylated DNA regulatory sequences, more preferably still at least six methylated DNA regulatory sequences, and ideally between at least seven, eight, nine, ten, eleven, twelve, thirteen, fourteen, fifteen, or sixteen methylated DNA regulatory sequences. Preferably, the at least one, two, three, four, five, six, seven, eight, nine, ten, eleven, twelve, thirteen, fourteen, fifteen, or sixteen methylated DNA regulatory sequences selected are combined with PSA.

Typically, the biological fluid is urine, or a derivative of urine following centrifugation or filtration, such as urine cell sediment.

In one aspect of the invention, there is provided, as set out in the appended claims, a method of determining the risk of aggressive prostate cancer in an individual diagnosed with prostate cancer, the method comprising a step of assaying a biological sample obtained from the individual for the presence of at last one methylated regulatory DNA sequence selected from group comprising: SEQ ID No's 1 to 16, or variants thereof, and correlating the presence or absence of the methylated regulatory DNA sequence with aggressive prostate cancer.

In one embodiment, detection of least three methylated regulatory DNA sequence from the group comprising SEQ ID NOs 1 to 16, or variants thereof, correlates with the presence of an aggressive prostate cancer.

In one embodiment, detection of least four, five or six methylated regulatory DNA sequence from the group comprising SEQ ID NOs 1 to 16, or variants thereof, correlates with the presence of aggressive prostate cancer.

Preferably, detection of six methylated regulatory DNA sequences as defined by SEQ ID NOs 1 to 6, or variants thereof, and having an AUC score of at least 80% correlates with the presence of an aggressive prostate cancer.

In one embodiment, the method further comprises detecting the presence of PSA in the biological sample.

In one embodiment, the sample is urine or a urine derivative from the individual.

In one embodiment, there is provided an assay of identifying an aggressive prostate cancer in an individual, the assay comprising the step of assaying a biological sample obtained from the individual for the presence of a methylated regulatory DNA sequence selected from group comprising: SEQ ID No's 1 to 16, or variants thereof, and at least one sequence selected from SEQ ID NOs 17 and 18, wherein detection of at least three of the methylated regulatory DNA sequences from SEQ ID NOs 1 to 16, or variants thereof, and one sequence from SEQ ID NOs 17 and 18 in a sample is indicative of the presence of an aggressive prostate cancer, and wherein the sensitivity of the assay for detecting the at least three methylated regulatory DNA sequences from SEQ ID NOs 1 to 16, or variants thereof, is at least 80%.

In one embodiment, the at least one sequence selected from SEQ ID NOs 17 and 18 is SEQ ID NO: 18 prostate-specific antigen (PSA).

Preferably, detection of at least six methylated DNA regulatory sequences as defined by SEQ ID NOs 1 to 6, or variants thereof, and having an AUC score of at least 80% correlates with the presence of an aggressive (metastatic) prostate cancer.

In one embodiment, there is provided a kit for detecting the presence of prostate cancer in a sample from an individual, the kit comprising a control oligonucleotide as defined by SEQ ID NO 19, 20 or 21, or a variant thereof, and a set of oligonucleotides for detecting SEQ ID NOs 1 to 16, or variants thereof, wherein detection of at least three sequences from SEQ ID NOs 1 to 16, or variants thereof, in the sample is indicative of the presence of prostate cancer, and wherein the sensitivity of the assay for detecting the at least three sequences from SEQ ID NOs 1 to 16, or variants thereof, is at least 80%.

In one embodiment, the prostate cancer is an aggressive prostate cancer.

In one embodiment, the kit further comprises an oligonucleotide for detecting the presence of PSA.

In one embodiment, the set of oligonucleotides is defined by SEQ ID NOs. 22 to 72.

In one embodiment, the kit further comprises a support having at least one oligonucleotide selected from group SEQ ID No's 1 to 16 anchored thereon.

In one embodiment, the kit comprises a support having three, four, five, six, seven, eight, nine, ten, eleven, twelve, thirteen, fourteen, fifteen, or sixteen oligonucleotides anchored thereon selected from SEQ ID NOs. 1 to 16.

In one aspect of the invention, there is provided, as set out in the appended claims, a method of determining the risk of aggressive prostate cancer in an individual, the method comprising a step of assaying a biological sample obtained from the individual for the presence of at last one methylated regulatory DNA sequence selected from group comprising: SEQ ID No's 1 to 16, or variants thereof, and correlating the presence or absence of the methylated regulatory DNA sequence with aggressive prostate cancer.

In one embodiment, detection of least three methylated regulatory DNA sequence from the group comprising SEQ ID NOs 1 to 16, or variants thereof, correlates with the presence of an aggressive prostate cancer.

Preferably, detection of SEQ ID NO. 1 and SEQ ID NO. 3, or variants thereof, together with at least one further sequence selected from SEQ ID NOs. 2, 4, 5 and 6, or variants thereof, correlates with the presence of an aggressive prostate cancer.

In one embodiment, detection of least four, five or six methylated regulatory DNA sequence from the group comprising SEQ ID NOs 1 to 16, or variants thereof, correlates with the presence of aggressive prostate cancer.

Preferably, detection of six methylated regulatory DNA sequences as defined by SEQ ID NOs 1 to 6, or variants thereof, and having an AUC score of at least 80% correlates with the presence of an aggressive prostate cancer.

In one aspect of the invention, there is provided, as set out in the appended claims, a method of detecting the presence of prostate cancer in an individual, the method comprising a step of assaying a biological sample obtained from the individual for the presence of at last one methylated regulatory DNA sequence selected from group comprising: SEQ ID No's 1 to 16, or variants thereof, and correlating the presence or absence of the methylated regulatory DNA sequence with a reference abundance marker indicative of prostate cancer.

In one aspect of the invention, there is provided, as set out in the appended claims, a method of determining whether an individual requires an invasive trans-rectal biopsy to confirm diagnosis of prostate cancer by histological review of a biopsy specimen, the method comprising a step of assaying a biological sample obtained from the individual for the presence of at last one methylated regulatory DNA sequence selected from group comprising: SEQ ID No's 1 to 16, or variants thereof, and in which the presence of the methylated regulatory DNA sequence determines that the individual requires an invasive trans-rectal biopsy to confirm diagnosis of prostate cancer by histological review of a biopsy specimen.

In one embodiment, the biological sample is urine or a urine derivative from the individual.

In this specification, the term "biological sample" or "biological fluid" may be a sample obtained from an individual such as, for example, urine or urine cell-sediment, blood or a prostate tissue sample from a biopsy or a radical prostatectomy. In many cases, the individual will be a person suspected of having prostate cancer, or pre-disposed to developing prostate cancer as determined by other phenotypic, genotypic or hereditary traits.

In this specification, the term "prostate cancer status" when used with reference to an individual primarily refers to the risk of the individual having the cancer. Depending on the number of biomarkers detected in the individual, the assay and methods of the invention will assist a clinician is determining the risk that the individual is positive for prostate cancer. Thus, in one embodiment, the methods, assays and kits of the invention provide a means for screening male patients to identify those patients that should undergo further investigative procedures, such as a biopsy. However, the term also encompasses prognostic evaluation of the cancer, identification of predisposition to developing the cancer, staging of the cancer, and evaluation or monitoring of the progress of the cancer, in the individual. The latter evaluation is typically employed as a means of monitoring the effectiveness of a treatment for the cancer.

A "variant" of one of SEQUENCE ID No's 1 to 16 shall be taken to mean at least 70% sequence identity, preferably at least 80% sequence identity, more preferably at least 90% sequence identity, and ideally at least 95%, 96%, 97%, 98% or 99% sequence identity with the native sequence.

mRNA expression of the KLK3 gene (positive control 2 - SEQ ID NO. 18) may be measured by any suitable method including, but not limited to, a Northern Blot or detection by hybridisation to a oligonucleotide probe. A variety of hybridization assays using a variety of technologies for hybridization and detection are available. For example, a TaqMan assay (PE Biosystems, Foster City, CA; See e.g., U.S. Patent Nos. 5,962,233 and 5,538,848,) is utilized. The assay is performed during a PCR reaction. The TaqMan assay exploits the 5'-3' exonuclease activity of the AMPLITAQ GOLD DNA polymerase. A probe consisting of an oligonucleotide with a 5 '-reporter dye (e.g., a fluorescent dye) and a 3 '-quencher dye is included in the PCR reaction. During PCR, if the probe is bound to its target, the 5'-3' nucleolytic activity of the AMPLITAQ GOLD polymerase cleaves the probe between the reporter and the quencher dye. The separation of the reporter dye from the quencher dye results in an increase of fluorescence. The signal accumulates with each cycle of PCR and can be monitored with a fluorometer.

DNA methylation may be measured by any suitable method, such as quantitative methylation specific PCR (PMID: 10734209).

In other embodiments, reverse-transcriptase PCR (RT-PCR) is used to detect the expression of RNA where RNA is enzymatically converted to complementary DNA or "cDNA" using a reverse transcriptase enzyme. The cDNA is then used as a template for a PCR reaction. PCR products can be detected by any suitable method, including but not limited to, gel electrophoresis and staining with a DNA specific stain or hybridization to a labelled probe. In some embodiments, the quantitative reverse transcriptase PCR with standardized mixtures of competitive templates method described in U.S. Patents 5,639,606, 5,643,765, and 5,876,978 is utilized.

In the specification, the term "high-risk prostate cancer", "high-risk disease" or "aggressive prostate cancer" or "metastatic prostate cancer" should be understood mean a prostate cancer that is categorised by the D'amico Risk Stratification criteria. The D'amico criteria are used to define low, intermediate and high-risk prostate cancer. For example, (i) Low risk: having a PSA less than or equal to 10, a Gleason score less than or equal to 6, or are in clinical stage T1-2a; (ii) Intermediate risk: having a PSA between 10 and 20, a Gleason score of 7, or are in clinical stage T2b; and (iii) High-risk: having a PSA more than 20, a Gleason score equal or larger than 8, or are in clinical stage T2c-3a. The terms high-risk, aggressive and metastatic can be used interchangeably. The terms high-risk and aggressive describe a cancer of high tumour grade (according to the Gleason scale, >=8) and a highly likelihood of metastasising.

In the specification, the term "gBlock®" should be understood to mean a doublestranded DNA molecule of 125-2000 bp in length. In this instance, the gBlock® is defined by SEQ ID NO. 19 and contains sequences for (A) an internal control ACTB, and the genes (B) GSTP1 (C), SFRP2, (D) IGFBP3, (E) IGFBP7, (F) APC and (G) PTGS2. The gBock® defined by SEQ ID NO: 20 was designed to contain the nucleotide sequences of bisulfite converted fully methylated internal control sequence *(ACTB)* and seven DNA regulatory sequences (*LXN, MAGPIE-1, DNAH10, ZMIZ1, CENPV* and *OR2L13).* The gBlock® defined by SEQ ID NO. 21 was designed to contain the nucleotide sequences of bisulfite converted fully methylated internal control sequence (*ACTB*) and four DNA regulatory sequences (*MTMR8, F3, CDH8 and*

*GALNTL6*).

Some of the uses of the invention include:

- To test for the presence of prostate cancer.
- Use as a novel screening test for any male at risk of having prostate cancer.
- Use as a non-invasive test using urine to determine which male requires an invasive trans-rectal biopsy to confirm a diagnosis of prostate cancer by histological review of a biopsy specimen.
- The test can be carried out on any biological sample that harbours prostate DNA, including blood plasma/serum, prostate tissue and metastatic lesions, either visceral or bone.

Some of the advantages of the invention is to:

- Reduce/eliminate unnecessary invasive biopsies in men who don't need them;
- Alleviate over-treatment of low-risk disease;
- Inform the clinician about the molecular biology of the disease; and
- Aid risk-stratification for selection of subsequent treatments/active surveillance.

## Brief Description of the Figures

The invention will be more clearly understood from the following description of an embodiment thereof, given by way of example only, with reference to the accompanying drawings, in which:-

**Figure 1** illustrates Feasibility Study data (n=156). Results on a panel of 156 pre-biopsy urine samples, shown by biopsy outcome. Positive-biopsy men are further categorised into low (LR), intermediate (IR) and high (HR) risk groups using the CAPRA score. Each row represents a gene, each column represents a patient. Methylation is measured as a continuous variable from 0-1000. Black squares indicate high methylation with a normalised index of methylation (NIM)>1, white squares indicate NIM=0 and shades of grey indicate intermediate NIM. A) discrimination between men with a positive biopsy versus men with a negative biopsy B) Selective detection of high-risk disease and C) selective detection of high-grade disease

**Figure 2** illustrates results reduced into categorical results: men with methylation of at least one gene and men with an NIM>1 in any one gene.

**Figure 3** illustrates ROC curves for (A) PSA alone, (B)-(C) using the invention, and (D) the test of the invention and PSA>4 ng/ml, which achieve an AUC of 0.54, 0.87 (average) and 0.96, respectively.

**Figure 4** illustrates that the positive predictive value of invention which indicates its utility for reducing number of unnecessary biopsies by selectively detecting aggressive PCa.

**Figure 5** illustrates standard curves constructed over a 6-log range using 5 independent qMSP measurements of a gBlock® fragment containing sequences for (A) internal control, (B) GSTP1 (C), SFRP2, (D) IGFBP3, (E) IGFBP7, (F) APC and (G) PTGS2 (SEQ ID NO. 19). Each qMSP assay has a slope of -3.3 (=/- 10%) and an $R^2$ >0.99, indicating a PCR efficiency close to 100%.

**Figure 6** illustrates graphs showing quantitative methylation-specific PCR data for SEQ ID NOs 7-16 in a radical prostatectomy cohort reveal quantitatively higher levels of DNA methylation in aggressive tumours (PC-A) compared with significant (PC-S) and indolent (PC-I) tumours and benign tissue.

## Detailed Description

*Materials and Methods*

Statistical methods:

Logistic regression is a standard method for modelling the relationship between a binary variable, in this case high-risk versus low-risk prostate cancer, and a set of continuous or categorical variables. For this analysis, the variables used for prediction consist of gene methylation values, as well as patient variables age and PSA. Mathematically this relationship is expressed as

$$\log\left(\frac{p_i}{1-p_i}\right) = \beta_1 X_{1i} + \beta_2 X_{2i} + \cdots + \beta_m X_{mi} \qquad (1)$$

where $p_i$ is the probability that the nth patient is high-risk based on their methylation profile and clinical characteristics, which are represented by the $X_{mi}$'s. The $\beta_m$ coeffcicents give the effect that each incremental change in methylation, age or PSA has on the log-odds of the patient being high risk of prostate metastasis.

Due to the cost of collection of biomarkers, and the general principle that simpler models lead to more robust predictions, one aim of the analysis is to choose the smallest number of predictor variables, the $X_m$'s, which will yield the best performing prediction model.

A LASSO[1] logistic regression (discussed below), along with a standard logistic regression incorporating six genes were fit to the data. Logistic regression models for each of the separate genes were also fitted for comparison. All models were trained using repeated 5-fold cross-validation with bootstrap resampling. The optimal cut-off value for prediction was then chosen using the entire data set.

A common problem with building a prediction model on the entire dataset is that the model will tend to over fit the current data set and will then underperform when new data is predicted from the model[7]. In general, more complex models will tend to adapt to the training data and will not generalise as well to new data. Therefore sparser models are preferred.

In an ideal case a model is fitted to some training data and then its performance is estimated on an independent test set. A model can be selected by choosing the model that performs best on the test dataset which consists of new unseen observations. For small datasets, a single split of the data into testing and training sets is often not possible.

Cross-validation is a method for performing multiple random training-test splits of a dataset. The process is as follows:

1. Split the data into K equally sized portions (K is usually chosen to be between 5 and 10);
2. Leave aside one of the K portions and train the model(s) on the all of the remaining K-1 portions together;

3. Test the performance of the model(s) on the portion of the data that was set aside in step 2; and
4. Repeat iteratively leaving out each of the K portions in succession.

This method gives a more accurate assessment of the out-of-sample prediction performance of the models than simply fitting the model to the entire dataset. To further account for uncertainty in the cross-validation process, the dataset is bootstrapped, *i.e.* resample the entire dataset with replacement, and perform cross-validation on each bootstrap iteration. For the analysis here, K= 5 and 2000 bootstrap iterations were used.

All of the data is used in the model-building and assessment stages. The model building process used aims to mitigate against any optimistic bias. Due to the relatively small number of high-risk cases, splitting the data is not an efficient option. Ideally a test or hold-out set of data which were not used in the training step should be used on which to test performance. This should be done at a later stage using an independently collected test data set.

The LASSO is a penalised regression method for building prediction models which mitigates against over-fitting on a data set. The LASSO is a method for both model selection and estimation. In standard logistic regression, the model parameters are fitted using iterative maximum likelihood. This estimates the parameters which fit the data the best, and as such can over-fit to the training data. Penalised regression methods add a penalty term to the estimation equation which penalises large values of the coefficients. This is a form of shrinkage which can yield more robust results. In the case of LASSO, the penalty term shrinks some coefficients to zero, acting as a form of variable selection.

The strength of penalisation is determined by a parameter, $\lambda$. The optimal lambda value is found by running a further cross-validation iteration within each iteration of the outer cross-validation loop.

For the final assessment of the LASSO model, models chosen in the resampling and cross-validation iterations were searched and selected as the final model, the most frequently occurring model. The performance assessments and model parameters were then based on the iterations where this model occurred. Regression coefficients were then obtained by averaging over these iterations.

<u>Samples</u>

The epiCaPture test (method described herein) is performed on urine or urine cell-sediment. The urine cell-sediment is obtained by centrifugation (10,000xg for 10 minutes) of a first-void urine sample (up to 50ml) following a digital rectal examination (DRE). The DRE consists of three strokes per lobe of the prostate gland. Enough pressure is applied to the prostate to depress the surface approximately 1cm, from the base toe the apex and from the lateral to the median line for each lobe. DNA is extracted from the cell sediment using a standard silica-membrane based extraction protocol (using a Qiagen total nucleic acid isolation kit, or similar commercially available product). Purified DNA (100ng) is subject to bisulfite conversion (using a Qiagen epitect kit, or similar commercially available product).

A 648 bp synthetic gBlock® DNA sequence (IDT - SEQ ID NO: 19) was designed to contain the nucleotide sequences of bisulfite converted fully methylated internal control sequence (*ACTB*) and six DNA regulatory sequences (*GSTP1, SFRP2, IGFBP3, IGFBP7, APC, PTGS2*):

AAAGGTGGAGGTAGTTAGGGTTTATTTGTATATTGATTTGAGATTAGTTGAATAAA
AGTGTATATTTTAAAAATGAGGTTAAGTGTGATTTTGTGGTGTGGAAAGTTGCGCG
GCGATTTCGGGGATTTTAGGGCGTTTTTTTGCGGTCGACGTTCGGGGTGTAGCGGTC
GTCGGGGTTGGGGTCGGCGGGAGTTCGAAAAGTTTTTCGGAGTTGCGCGCGGGTTT
GTAGCGTTTCGTTCGCGTTGTTTTTTCGGTGTTTCGTTTTTTCGCGTTTTAGTCGTCG
GTTGTTAGTTTTTCGGGGTTTCGAGTCGTATTTAGCGAAGAGAGCAAATTTTTTCGA
TATCGGTTCGTCGTAGGGAGATTTTATTTCGAGAGCGGAAGGGGTAAGGGCGGCGG
GGTTAAGGAGATCAAAAAGCGGGCGTGAGATCGAGCGTTTATGGGTCGGTTACGTC
GGGTGTTCGTTTATTTTTCGACGTTAGTAGGAGCGCGAAATTATATGTCGGTTACGT
GCGTTTATATTTAGTTAATCGGCGGGTTTTCGACGGGAATGGGGAGCGTTTTGGTTC
AAACGGAAGCGTTCGGGTAAAGATTGCGAAGAAGAAAAGATATTTGGCGGAAATT
TGTGCGTTTGGGGCGGTGGAATTCAAA

An 885 bp synthetic gBlock® DNA sequence (IDT - SEQ ID NO: 20) was designed to contain the nucleotide sequences of bisulfite converted fully methylated internal control sequence (*ACTB*) and six DNA regulatory sequences (*LXN, MAGPIE-1, DNAH10, ZMIZ1, CENPV and OR2L13*):

AAAGGTGGAGGTAGTTAGGGTTTATTTGTATATTGATTTGAGATTAGTTGAATAAA
AGTGTATATTTTAAAAATGAGGTTAAGTGTGATTTTGTGGTGTGGAAATGGGTTATT
TTGGTTTAACGGGATTAGTAGTAGAGCGTCGTTCGTTTTGTTTGTTGTTGGGTTCGG
TTGTCGAGGCGGAAAAGTCGTAAGAAATTTGTTTTTGGTTTTTGTAGGCGTTTGGGT
TGTTTTATTTGAGAGTTGCGTAGGGCGGTTTGGCGGTGGTTGTTGTTTATATAATTC
GAAACGTCGAGGTGTTGTGATTTTCGTTTCGTTAATTTTTTAGTTTTAGTTTTATTTG
TAAGGTGGGCGGGTTGTTTGTAAATCGGTCGGCGTGGGGTGGGGTCGTATTTTCGG
TTGTAGCGGTTAAAGGGTTTCGTCGTCGTTTTCGTTCGGAGGTTGGAGTGTTGTTCG
TCGGGTCGTGCGTTCGTTCGGTAGCGGCGTGTATTAGTATTATAAACGTTGGGACGT

ATTTGTTTGTTTAGTTTCGTAGGTAGAGGGGTTGCGTTTGGGTTTACGTTCGCGATA
TTTAGAATTTATTCGTATTTGCGAAGGCGAAAATACGTTTTTGTCGGTCGTTTAGTTT
TTTGTAGGTGTAAGGGCGGATGTTTTAGCGATTACGGGAGTCGGGTTGGGGAGGTT
GGTGGGGGGCGGGGGGAGTTTTAAATTTAGGTTCGTTTAGTTATAGGCGTTTAGGTT
TAGTCGGAAATTGTCGGAGGACGCGTTGTTGCGAGATTAGTCGCGGCGTTTTTGGT
AGTAGTGGGCGTGTTTGCGGGTTTAGGAGGGTTTTTTTTCGCGATCGTCGATTACG
ATGAGAGCGTGAAGATTTTTTCGAAAGGAAAA

A 643 bp synthetic gBlock® DNA sequence (IDT - SEQ ID NO: 21) was designed to contain the nucleotide sequences of bisulfite converted fully methylated internal control sequence *(ACTB)* and four DNA regulatory sequences (*MTMR8, F3, CDH8 and GALNTL6*):

AAAGGGCGGTTTTGGTTTAAATTTTCGTTATTTGATTTTCGGCGAAAGTTTTTATCGC
GATATTTTGATCGTAGTCGTTTTCGTTAAAAAAGGTGGAGGTAGTTAGGGTTTATTT
GTATATTGATTTGAGATTAGTTGAATAAAAGTGTATATTTTAAAAATGAGGTTAAGT
GTGATTTTGTGGTGTGGAAATTCGGTTTATTACGGCGGTTATTTTTCGGGTAGTGAC
GACGATCGAGACGGTGAGGGCGGTTATCGTTGGGGAGGGAGGTTCGGGTTTAGGTT
TGGAAGTAAAACGTGTGGTTTGTTATATCGTCGGTTGTATTGGATTAGGATTATTTT
TTATGAAGGTTTGTTTTGTTAGTACGTAGTAGGTTTTAGTTTTTACGTCGTTTCGAAT
ATTTCGTAGAAATACGGGGTATGTATAACGAAACGGATTTCGTTTATGTTTTTCGGA
GTTATAGGGTTTGGTGCGGAGACGTAGGGCGGGCGCGTTGGGTTTTGGGTGTTCGT
AATTTAAAGTGTAGTTGGTGTAAACGGGTCGTTTTTATTTTTCGTAGTCGTCGCGTTT
CGGTTCGTCGCGTTTTCGTCGGTATTATGGAGGAATTTTTGGGATAATCGTTTGTAG
TCGGCGATTGGGAAA

**Table 1**: The gBlock® (SEQ ID NO. 19) was used to construct to-fold serial dilutions over a 6-log template concentration range to determine the dynamic range and PCR efficiency of each epiCaPture assay:

| Standard | Copy number | Vol. of gblock (μl) | Vol. of molecular grade $H_2O$ (μl) | DNA conc. (pg/μl) |
|---|---|---|---|---|
| 1 | 1,000,000 | 10 (WS) | 173.8 | 0.544 |
| 2 | 100,000 | 10 (1) | 90 | 0.0544 |
| 3 | 10,000 | 10 (2) | 90 | 0.00544 |
| 4 | 1,000 | 10 (3) | 90 | 0.000544 |
| 5 | 100 | 10 (4) | 90 | 0.0000544 |
| 6 | 10 | 10 (5) | 90 | 0.00000544 |

Quantitative methylation specific PCR (qMSP) is performed, as previously described [3-5]. The PCR efficiency of each of the assays (internal control and 6 targets) was rigorously evaluated by performing 5 independent replicates (each with 3 technical replicates) over a 6-log template concentration range (Figure 5). Bisulfite treated DNA is amplified in parallel TaqMan® PCR reactions performed with oligonucleotides specific for each of the target methylated DNA regulatory sequences (SEQ ID NOs 1 to 6) and the endogenous control gene *ACTB* (SEQ ID NO: 17). Samples are considered positively amplified when a comparative threshold cycle ($C_T$) of <50 was detected in at least two out of three replicates. A normalized index of methylation (NIM) was calculated, as previously described[6], to determine the ratio of the normalized amount of methylated target to the normalized amount of *ACTB* in any given sample, by applying the formula:

$$\text{NIM} = [(TARGET_{\text{ample}}/TARGET_{\text{MC}})/(ACTB_{\text{sample}}\ /ACTB_{\text{MC}})] \times 1000 \qquad (2)$$

Where $TARGET_{sample}$ is the quantity of fully methylated copies of each of the sequences being sampled in any individual sample, $\text{TARGET}_{MC}$ is the quantity of fully methylated copies of *each of the sequences being sampled* in a commercially available fully methylated bisulfite converted human DNA sample (Qiagen product number 59655), *ACTB* sample is the quantity of bisulfite modified templates in any individual sample and $ACTB_{MC}$ is the quantity of bisulfite modified templates in the universally methylated control DNA.

All genomic sequences for *GSTP1, SFRP2, IGFBP3, IGFBP7, APC, PTGS-2, LXN, MAGPIE-1, DNAH10, ZMIZ1, CENPV, OR2L13, MTMR8, F3, CDH8* and *GALNTL6* were obtained from the UCSC Human Genome Browser (http://genome-euro.ucsc.edu).

*Results*

Results from the study on 156 men (Figures 3(A) to 3(D)) demonstrate that the invention can non-invasively discriminate high-risk (metastatic) PCa from low-risk (less chance of metastasis) disease and benign enlargement of the prostate (AUC=0.86). In this cohort, a high score (NIM>1) had 100% specificity for PCa, and greatly outperformed PSA, which yielded a PCa-specificity of only 11.63%.

**Table 2** is data relating to the cohort of 156 TRUS-biopsy patients. The exclusion criteria for the cohort were (1) metastases on an MRI and/or a bone scan and (2) not post-DRE.

| | **Biopsy positive** | **Biopsy negative** | **P value** |
|---|---|---|---|
| N | 108 | 48 | |
| **Age (years)** | | | |
| Mean | 69.95 | 64.85 | 0.008 |
| Median | 69.50 | 66 | |
| Range | 53-85 | 42-82 | |

(continued)

| PSA (ng/ml) | | | |
|---|---|---|---|
| Mean | 16.53 | 7.06 | <0.0001 |
| Median | 10 | 6.10 | |
| Range | 4.1-95.9 | 0.2-30.30 | |
| **Risk group** | | | |
| LR | 14 | | |
| IR | 58 | | |
| HR | 36 | | |

Urinary detection of prostate cancer

For non-invasively distinguishing men who have prostate cancer form those who do not (or more strictly speaking, men with a positive biopsy from men with a negative biopsy), the best combination of biomarkers is *GSTP1* used in conjunction with *PSA*. This is calculated using a LASSO model (Table 3, Figure 3A). This achieved a positive predictive value (PPV) of 92%, with a negative predictive value (NPV) of 52%, with a sensitivity and specificity for prostate cancer of 81% and 77%, respectively. The combination of six methylated DNA regulatory sequences (as defined by SEQ ID NOs 1 to 6) in the method described herein also performs well at non-invasive detecting prostate cancer: PPV = 92%, NPV = 51%, sensitivity = 60% and specificity = 89%.

| **Table 3: Biopsy positive versus biopsy negative** | | | | | |
|---|---|---|---|---|---|
| **SEQ (Gene)** | AUC | Sensitivity | Specificity | PPV | NPV |
| **1 (GSTP1)** | 0.72 (0.66 - 0.77) | 0.44 | 0.98 | 0.98 | 0.45 |
| **2 (SFRP2)** | 0.66 (0.60 - 0.72) | 0.37 | 0.94 | 0.93 | 0.41 |
| **3 (IGFBP3)** | 0.65 (0.58 - 0.73) | 0.41 | 0.89 | 0.89 | 0.41 |
| **4 (IGFBP7)** | 0.64 (0.58 - 0.70) | 0.29 | 1 | 1 | 0.40 |
| **5 (APC)** | 0.68 (0.60 - 0.75) | 0.49 | 0.87 | 0.89 | 0.44 |
| **6 (PTGS2)** | 0.63 (0.55 - 0.72) | 0.34 | 0.98 | 0.97 | 0.41 |
| **PSA** | 0.76 (0.67 - 0.84) | 0.88 | 0.51 | 0.80 | 0.67 |
| **All** | 0.75 (0.68 - 0.81) | 0.60 | 0.89 | 0.92 | 0.51 |
| **LASSO (1 (GSTP1) + PSA)** | 0.83 (0.74 - 0.89) | 0.81 | 0.77 | 0.92 | 0.50 |

Urinary detection of high-risk prostate cancer

However, as stated already, the dilemma for prostate cancer detection is not in the ability to detect the entire spectrum of disease, for which PSA is already adequately doing, but to specifically detect high-risk disease with high likelihood to metastasise. For predicting high-risk prostate cancer according to D'Amico criteria[2], the LASSO, which is the selection method used here, determines that GSTP1 and IGFBP3 are the best fit (Table 4, Figure 3B). This combination delivers a PPV 56% of and NPV of 94% for high-risk disease, with a sensitivity and specificity both at 82%. The combination of all 6 genes, performs slightly less well, delivering a sensitivity of 52% and a specificity of 92%, for high risk disease. The method described herein (and derivations of it) outperforms current clinical practice (PSA), which in this cohort was found to have a sensitivity of 100% and specificity of only 21% (at the 4ng/ml cut-off) for high-risk disease.

| **Table 4. Detection of high-risk disease** | | | | | |
|---|---|---|---|---|---|
| **SEQ (Gene)** | AUC | Sensitivity | Specificity | PPV | NPV |
| **1 (GSTP1)** | 0.78 (0.68 - 0.87) | 0.70 | 0.87 | 0.61 | 0.92 |

(continued)

| Table 4. Detection of high-risk disease | | | | | |
|---|---|---|---|---|---|
| **SEQ (Gene)** | AUC | Sensitivity | Specificity | PPV | NPV |
| **2 (SFRP2)** | 0.77 (0.68 - 0.86) | 0.67 | 0.85 | 0.55 | 0.90 |
| **3 (IGFBP3)** | 0.76 (0.66 - 0.86) | 0.61 | 0.82 | 0.49 | 0.88 |
| **4 (IGFBP7)** | 0.77 (0.67 - 0.86) | 0.58 | 0.91 | 0.63 | 0.88 |
| **5 (APC)** | 0.76 (0.66 - 0.86) | 0.67 | 0.84 | 0.54 | 0.90 |
| **6 (PTGS2)** | 0.71 (0.60-0.82) | 0.52 | 0.92 | 0.63 | 0.87 |
| **All six** | 0.84 (0.75 - 0.93) | 0.79 | 0.82 | 0.55 | 0.93 |
| **LASSO (1 (GSTP1) + 3 (IGFBP3))** | 0.83 (0.75 - 0.92) | 0.82 | 0.82 | 0.56 | 0.94 |

Urinary detection of high-grade prostate cancer.

The Gleason grading system is the strongest prognostic indicator for prostate cancer. It is a histological grading system based on the glandular pattern of the tumour. A Gleason score is obtained by the addition of the primary and secondary grades. The presence of Gleason grade 4 or higher, or a Gleason score of 7 or higher predicts a poor prognosis.

For predicting tumours with a high Gleason score (>=8), the combination of all 6 biomarkers outlined above outperforms all biomarkers assessed individually (Table 5, Figure 3C), with a PPV of 48%, a NPV of 96% and a sensitivity and specificity of 76% and 87%, respectively. Combining all six markers with PSA gives some improvement again, with a sensitivity and specificity of 86% and 82%.

| Table 5: Detection of high-grade disease | | | | | |
|---|---|---|---|---|---|
| **SEQ (Gene)** | AUC | Sensitivity | Specificity | PPV | NPV |
| **1 (GSTP1)** | 0.74 (0.63 - 0.86) | 0.67 | 0.82 | 0.38 | 0.94 |
| **2 (SFRP2)** | 0.78 (0.67 - 0.89) | 0.67 | 0.88 | 0.48 | 0.94 |
| **3 (IGFBP3)** | 0.78 (0.67 - 0.90) | 0.67 | 0.82 | 0.38 | 0.94 |
| **4 (IGFBP7)** | 0.77 (0.66 - 0.89) | 0.71 | 0.78 | 0.35 | 0.94 |
| **5 (APC)** | 0.75 (0.62 - 0.87) | 0.71 | 0.80 | 0.38 | 0.94 |
| **6 (PTGS2)** | 0.68 (0.54 - 0.83) | 0.57 | 0.89 | 0.46 | 0.93 |
| **PSA** | 0.79(0.71-0.87) | 0.95 | 0.63 | 0.29 | 0.99 |
| **All 6** | 0.83 (0.73 - 0.94) | 0.76 | 0.87 | 0.48 | 0.96 |
| **All 6 + PSA** | 0.86 (0.76 - 0.96) | 0.86 | 0.82 | 0.44 | 0.97 |
| **LASSO (3 (IGFBP3))** | 0.78 (0.67 - 0.90) | 0.67 | 0.82 | 0.38 | 0.94 |

In the specification the terms "comprise, comprises, comprised and comprising" or any variation thereof and the terms "include, includes, included and including" or any variation thereof are considered to be totally interchangeable and they should all be afforded the widest possible interpretation and vice versa.

The invention is not limited to the embodiments hereinbefore described but may be varied in both construction and detail.

**References**

1. Tibshirani R. Regression shrinkage and selection via the LASSO. J. Royal. Statist. Soc B., 1996 vol. 58(1): 267-288.
2. Bastian PJ, Boorjian SA, Bossi A, et al. High-risk prostate cancer: from definition to contemporary management. European urology 2012; 61(6): 1096-106.

3. Eads CA DK, Kawakami K, Saltz LB, Blake C, Shibata D, Danenberg PV, Laird PW. MethyLight: a high-throughput assay to measure DNA methylation. Nucleic Acids Research 2000; 28(8): E32.
4. Perry AS, Loftus B, Moroose R, et al. In silico mining identifies IGFBP3 as a novel target of methylation in prostate cancer. British journal of cancer 2007; 96(10): 1587-94.
5. Perry AS, O'Hurley G, Raheem OA, et al. Gene expression and epigenetic discovery screen reveal methylation of SFRP2 in prostate cancer. International journal of cancer Journal international du cancer 2013; 132(8): 1771-80.
6. Yegnasubramanian S, Kowalski J, Gonzalgo ML, et al. Hypermethylation of CpG islands in primary and metastatic human prostate cancer. Cancer Res 2004; 64(6): 1975-86.
7. Hastie TaF. The elements of statistical learning. Springer-Verlag 2009; 2nd edition.

**Claims**

1. A method of determining the risk of aggressive prostate cancer in an individual diagnosed with prostate cancer, the method comprising a step of assaying a biological sample obtained from the individual for the presence of at last one methylated regulatory DNA sequence selected from group comprising: SEQ ID No's 1 to 16, or variants thereof, and correlating the presence or absence of the methylated regulatory DNA sequence with aggressive prostate cancer.

2. A method according to Claim 1, wherein detection of least three methylated regulatory DNA sequence from the group comprising SEQ ID NOs 1 to 16, or variants thereof, correlates with the presence of an aggressive prostate cancer.

3. A method according to Claim 1 or Claim 2, wherein detection of least four, five or six methylated regulatory DNA sequence from the group comprising SEQ ID NOs 1 to 16, or variants thereof, correlates with the presence of aggressive prostate cancer.

4. A method according to Claim 1, wherein detection of six methylated regulatory DNA sequences as defined by SEQ ID NOs 1 to 6, or variants thereof, and having an AUC score of at least 80% correlates with the presence of an aggressive prostate cancer.

5. A method according to any one of the preceding claims in which the sample is urine or a urine derivative from the individual.

6. An assay of identifying an aggressive prostate cancer in an individual, the assay comprising the step of assaying a biological sample obtained from the individual for the presence of a methylated regulatory DNA sequence selected from group comprising: SEQ ID No's 1 to 16, or variants thereof, and at least one sequence selected from SEQ ID NOs 17 and 18, wherein detection of at least three of the methylated regulatory DNA sequences from SEQ ID NOs

1 to 16, or variants thereof, and one sequence from SEQ ID NOs 17 and 18 in a sample is indicative of the presence of an aggressive prostate cancer, and wherein the sensitivity of the assay for detecting the at least three methylated regulatory DNA sequences from SEQ ID NOs 1 to 16, or variants thereof, is at least 80%.

**7.** An assay according to Claim 6, in which the at least one sequence selected from SEQ ID NOs 17 and 18 is SEQ ID NO: 18 prostate-specific antigen (PSA).

**8.** An assay according to Claim 7 or Claim 8, in which detection of at least six methylated DNA regulatory sequences as defined by SEQ ID NOs 1 to 6, or variants thereof, and having an AUC score of at least 80% correlates with the presence of an aggressive (metastatic) prostate cancer.

**9.** A kit for detecting the presence of prostate cancer in a sample from an individual, the kit comprising a control oligonucleotide as defined by SEQ ID NO 19, 20 or 21, or a variant thereof, and a set of oligonucleotides for detecting SEQ ID NOs 1 to 16, or variants thereof, wherein detection of at least three sequences from SEQ ID NOs 1 to 16, or variants thereof, in the sample is indicative of the presence of prostate cancer, and wherein the sensitivity of the assay for detecting the at least three sequences from SEQ ID NOs 1 to 16, or variants thereof, is at least 80%.

**10.** A kit as claimed in Claim 9, in which the prostate cancer is an aggressive prostate cancer.

**11.** A kit as claimed in Claim 9 or Claim 10, wherein the kit further comprises an oligonucleotide for detecting the presence of PSA.

**12.** A kit as claimed in any one of Claims 9 to 11, wherein the set of oligonucleotides is defined by SEQ ID NOs. 22 to 72.

**13.** A kit as claimed in any one of Claims 9 to 12, wherein the kit further comprises a support having at least one oligonucleotide selected from group SEQ ID No's 1 to 16 anchored thereon.

**14.** A kit as claimed in Claim 13, wherein the kit comprises a support having three, four, five, six, seven, eight, nine, ten, eleven, twelve, thirteen, fourteen, fifteen, or sixteen oligonucleotides anchored thereon selected from SEQ ID NOs. 1 to 16.

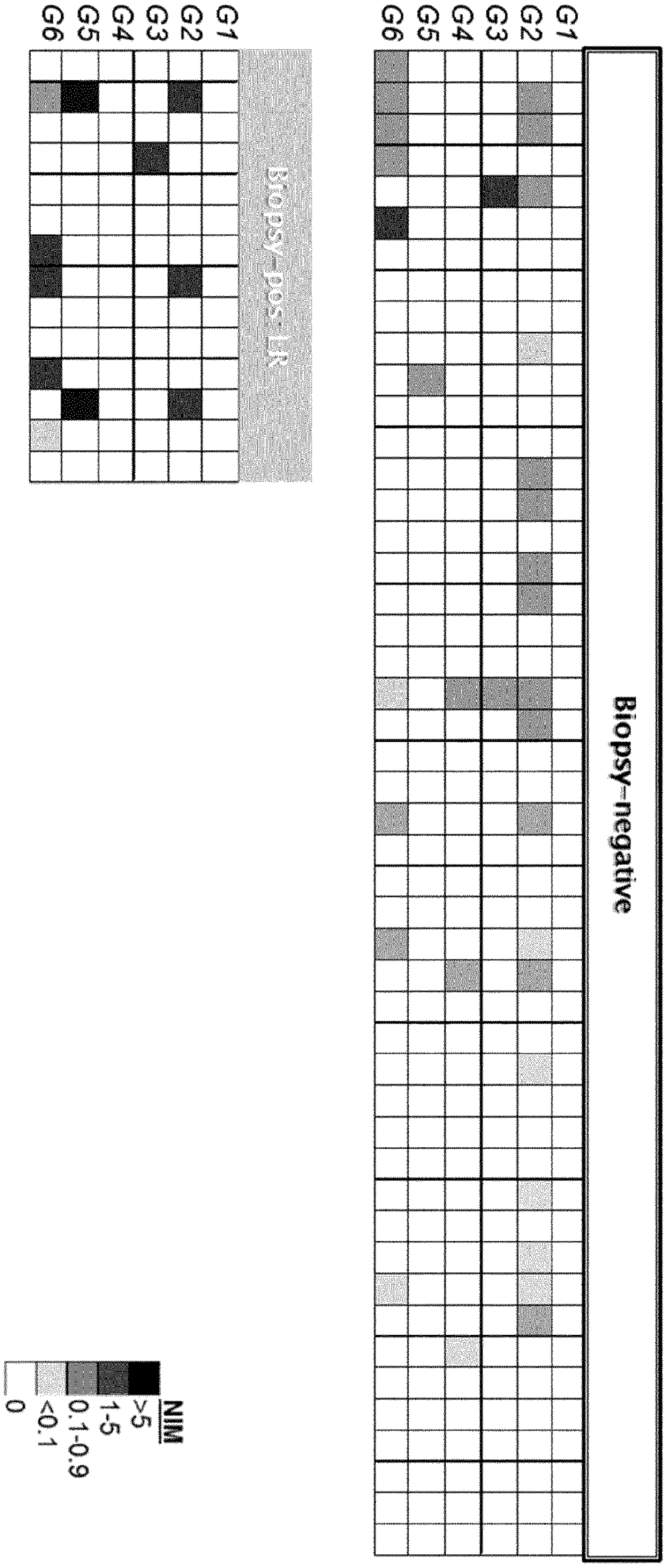
Biopsy-negative
Biopsy-pos LR
G1
G2
G3
G4
G5
G6
NIM
>5
1-5
0.1-0.9
<0.1
0

Figure 1

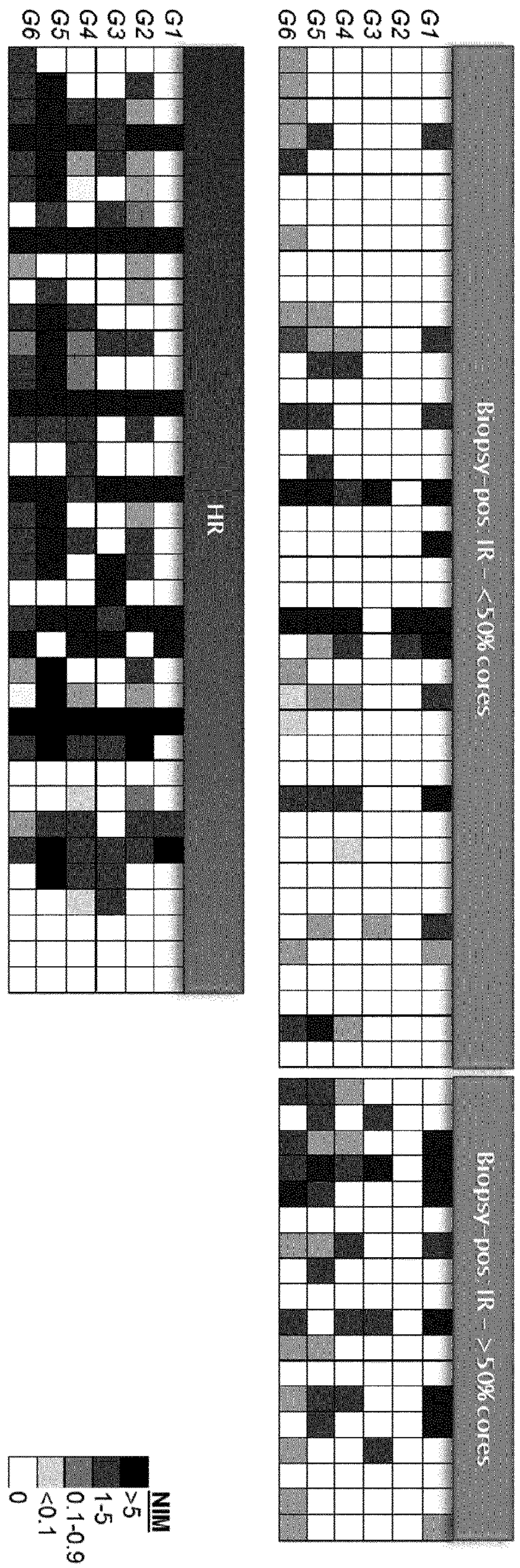


Figure 1 (continued)

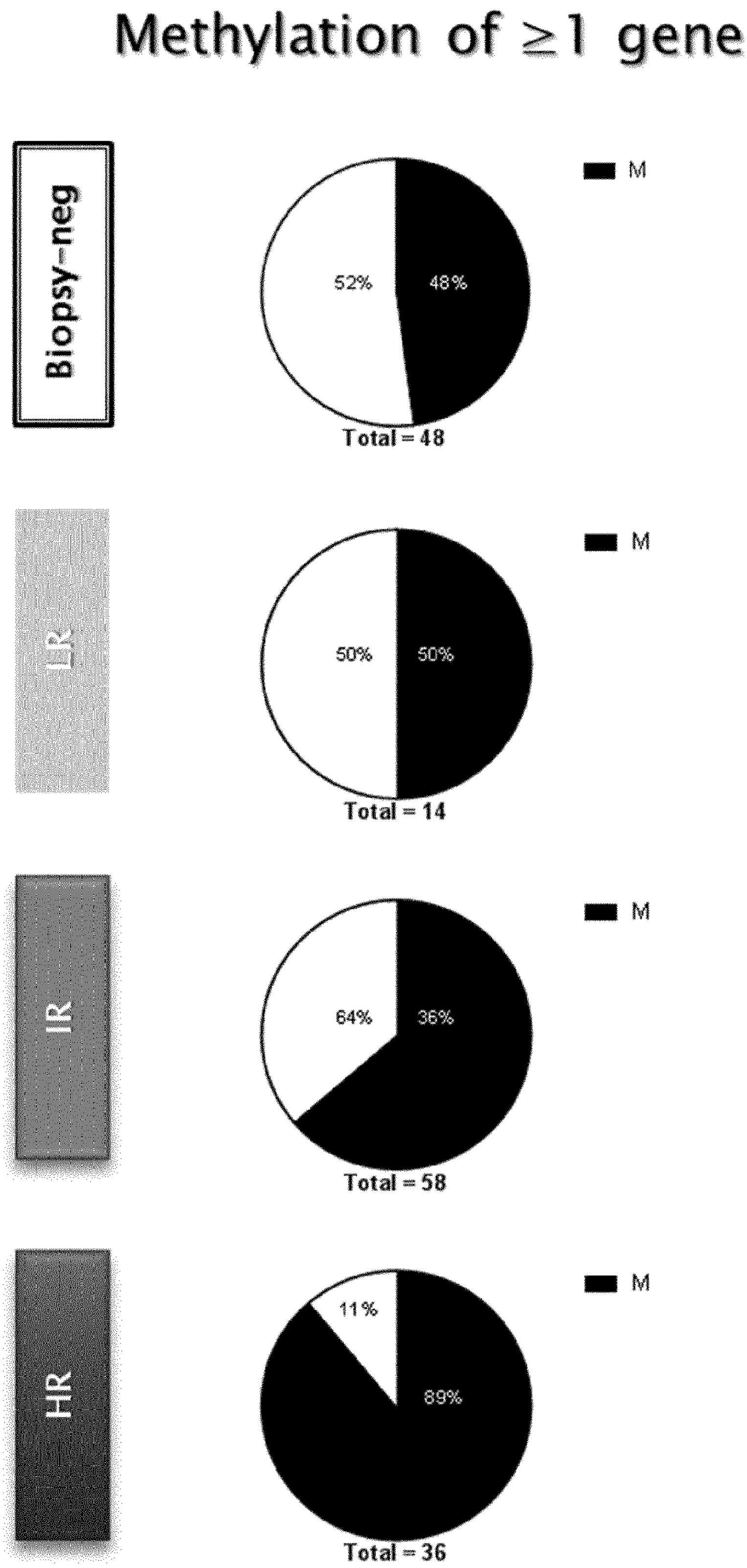
Methylation of ≥1 gene
Biopsy-neg
M
52%
48%
Total = 48
LR
M
50%
50%
Total = 14
IR
M
64%
36%
Total = 58
HR
M
11%
89%
Total = 36

Figure 2

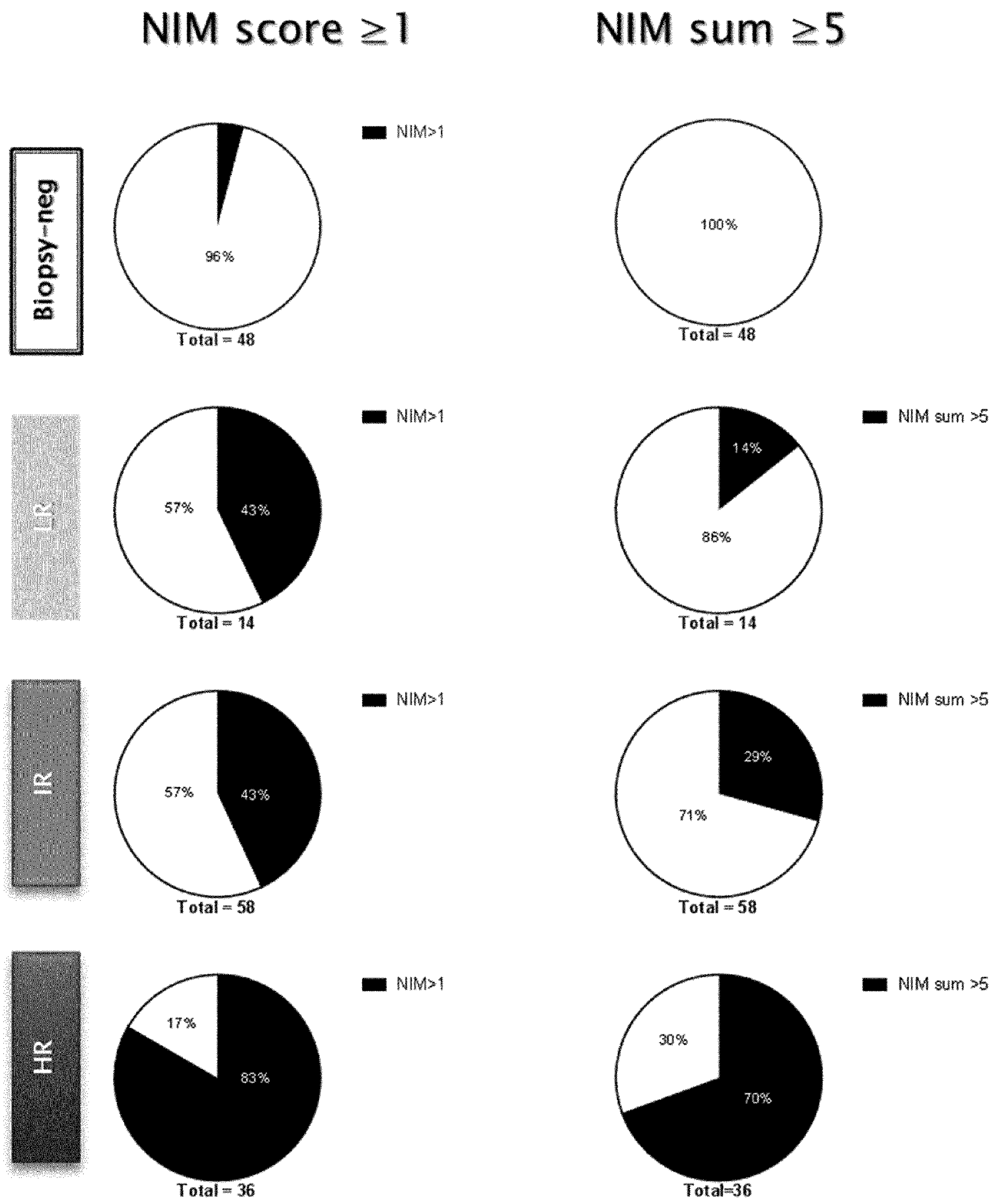


Figure 2 (cont.)

**(A) PSA**

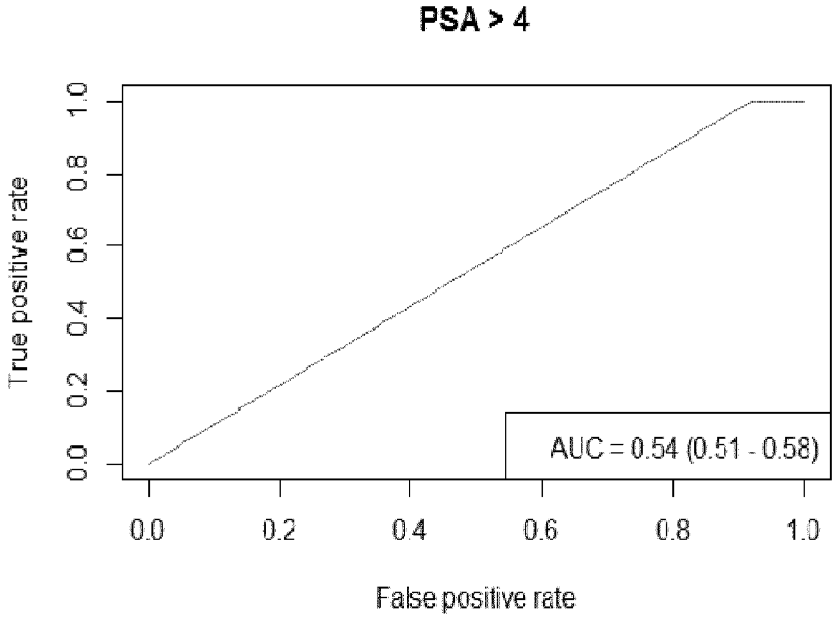


**(B) Individual Methylation Panel**

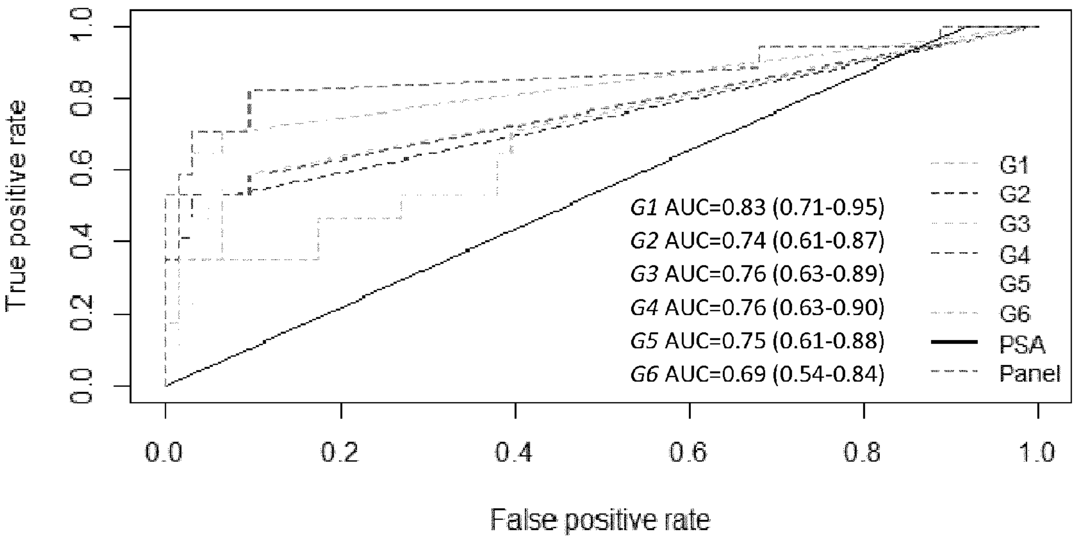


**Figure 3**

(C) **Methylation Panel**

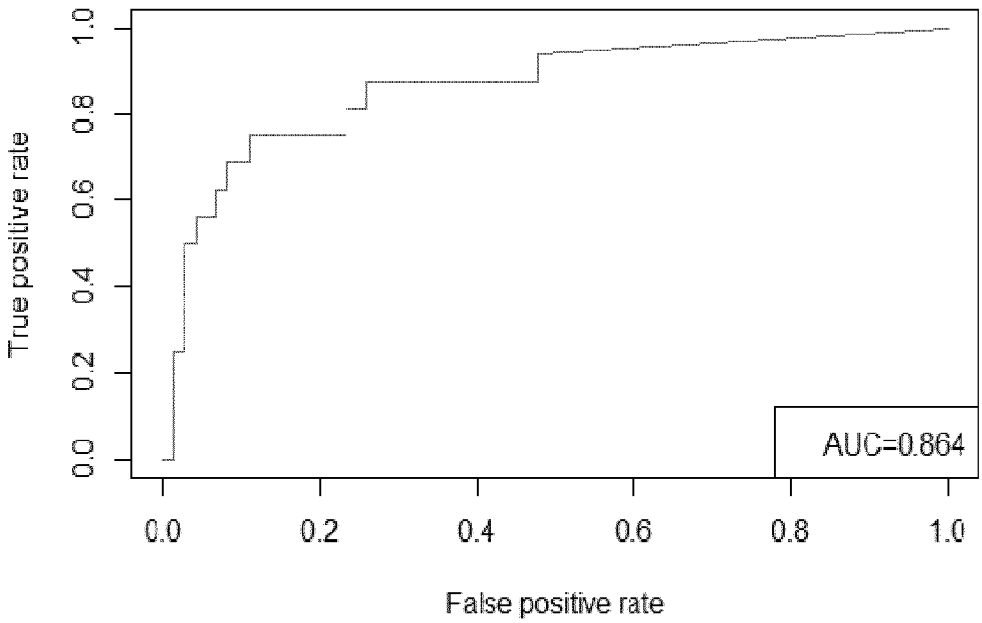


(D)

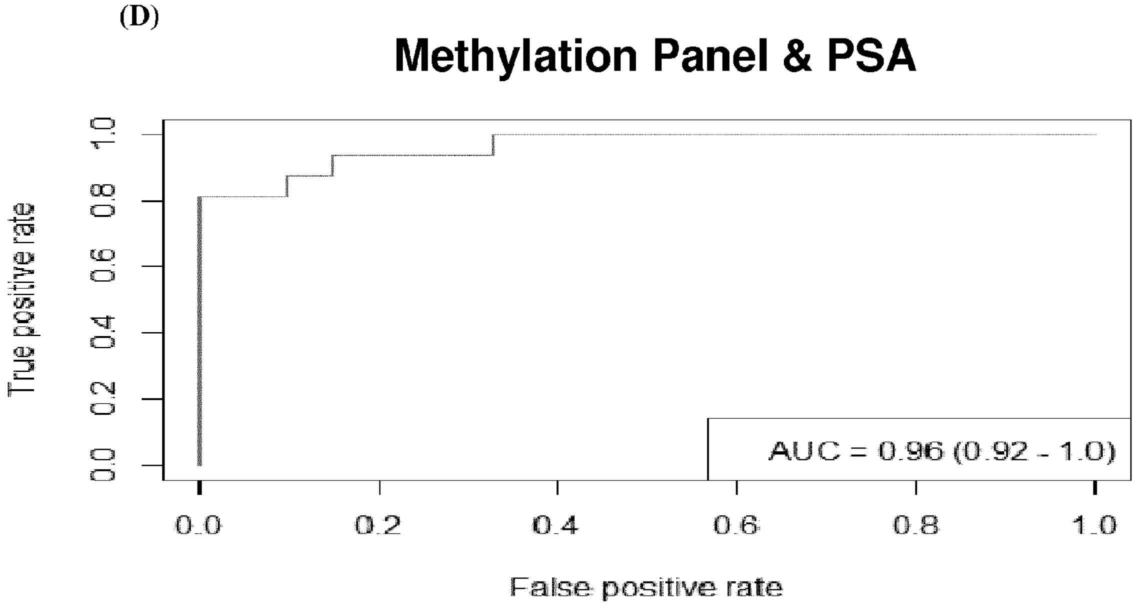


**Sensitivity = 0.94**
**Specificity = 0.67**

**Figure 3 (cont.)**

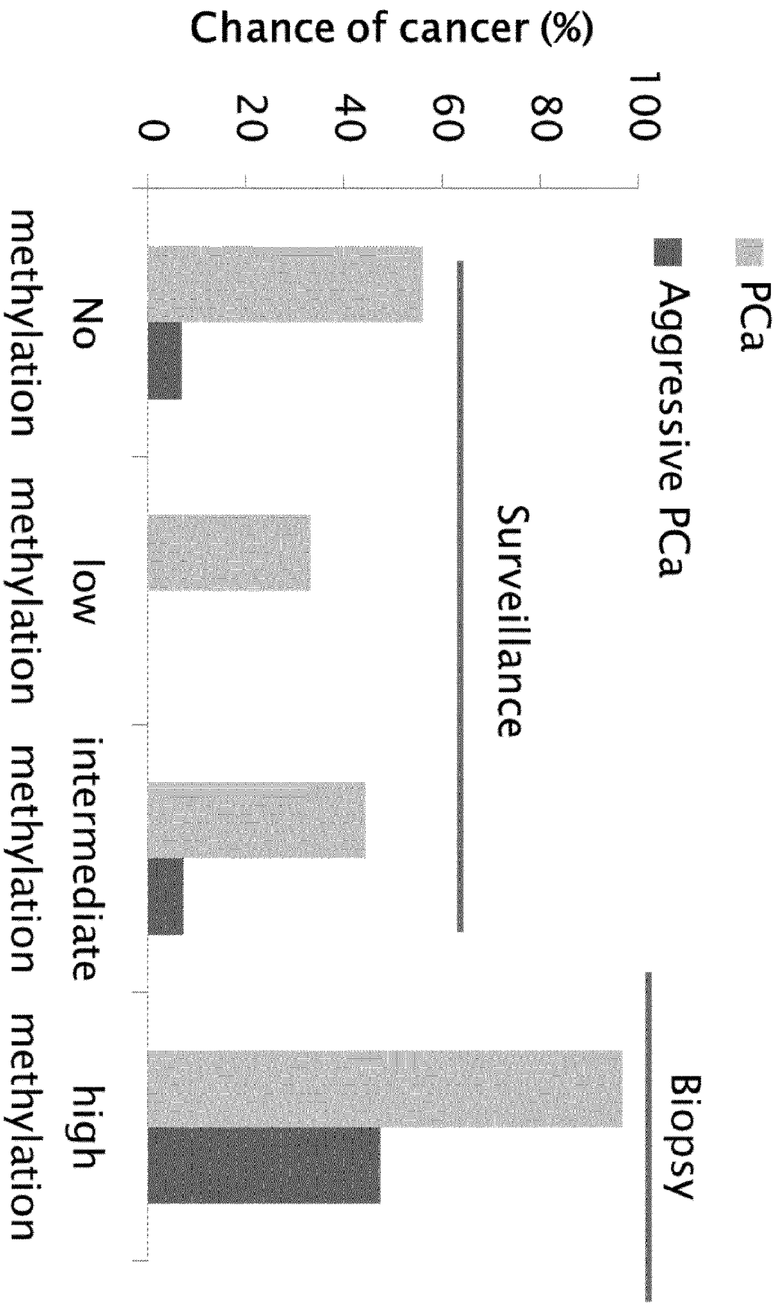
Chance of cancer (%)
0
20
40
60
80
100
PCa
Aggressive PCa
Surveillance
Biopsy
No methylation
low methylation
intermediate methylation
high methylation

Figure 4

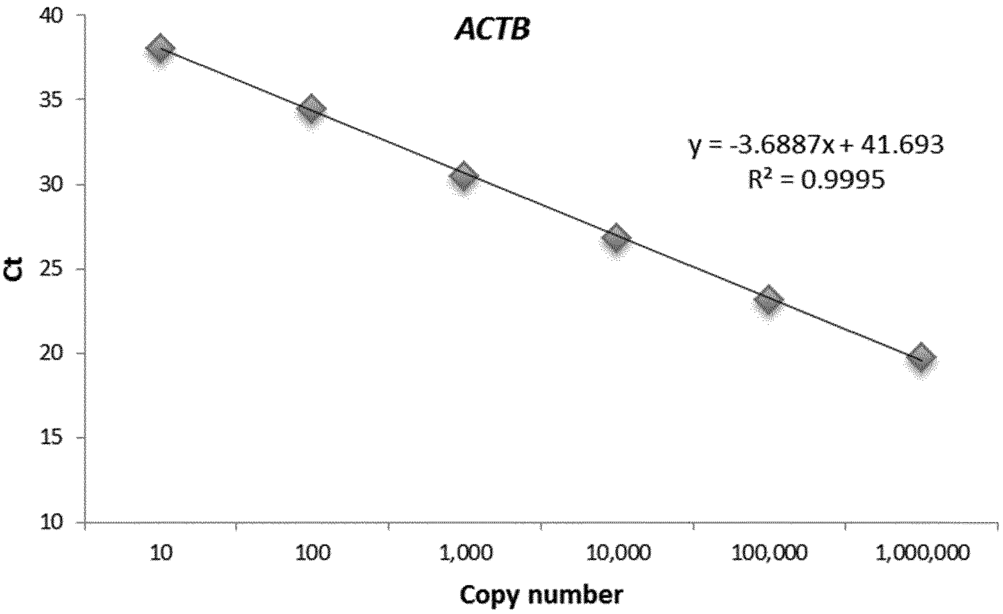
ACTB
y = -3.6887x + 41.693
R² = 0.9995
Ct
40
35
30
25
20
15
10
10
100
1,000
10,000
100,000
1,000,000
Copy number

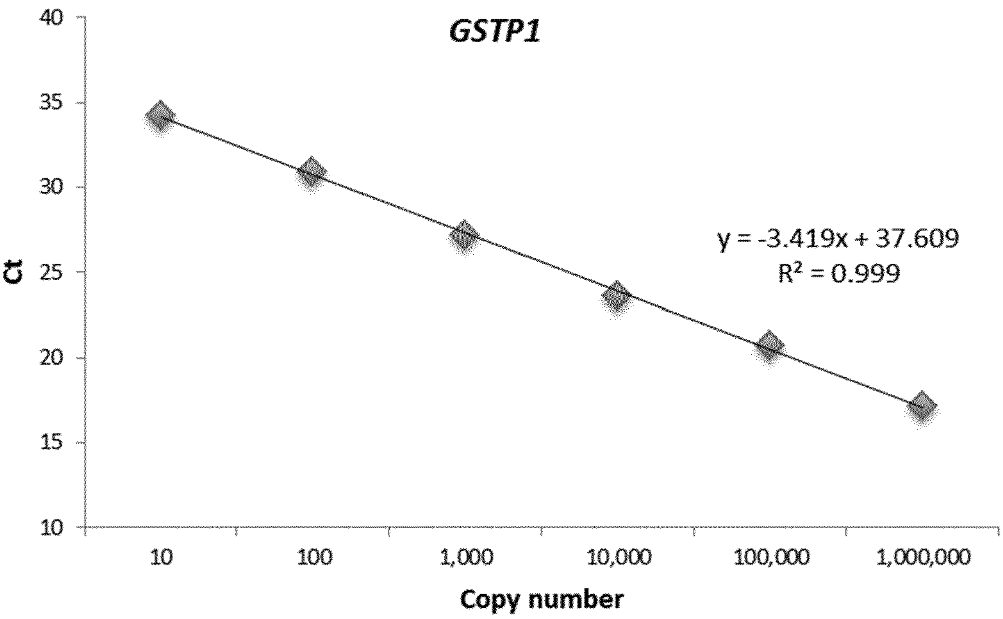
GSTP1
y = -3.419x + 37.609
R² = 0.999
Ct
40
35
30
25
20
15
10
10
100
1,000
10,000
100,000
1,000,000
Copy number

Figure 5

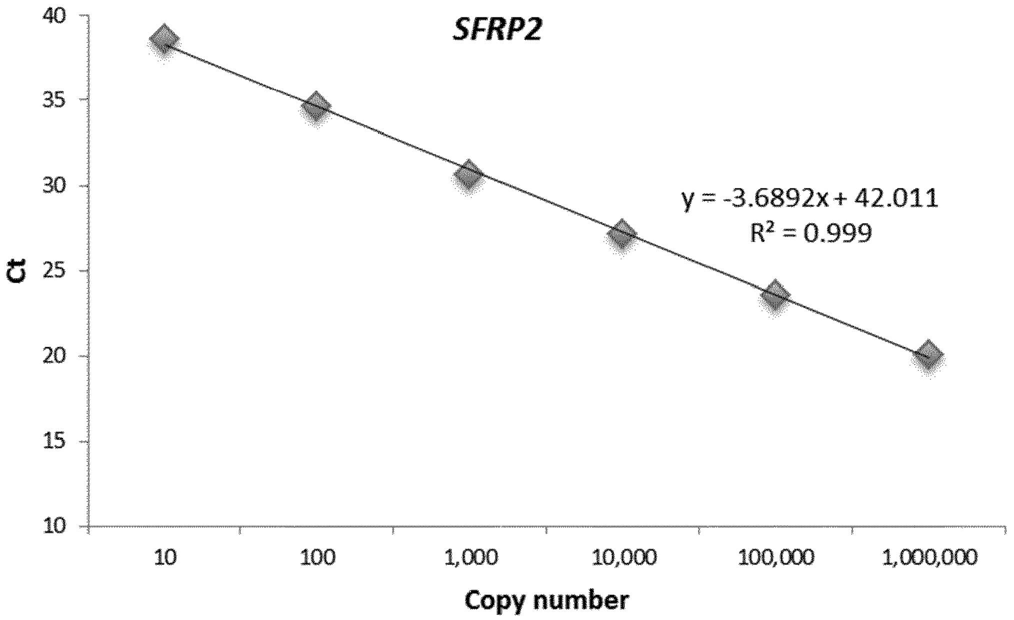


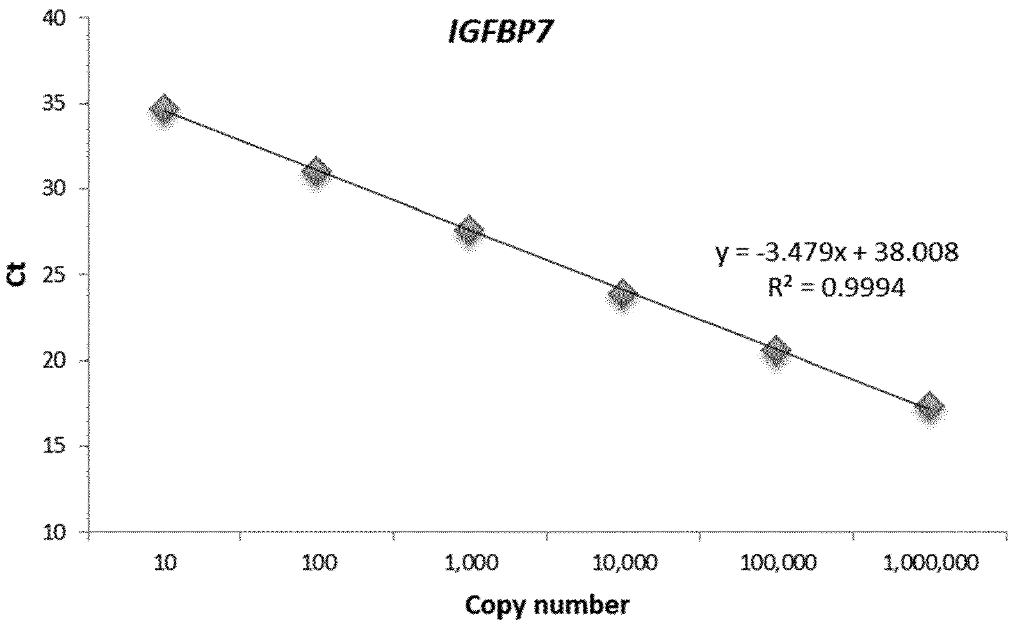


Figure 5 (cont.)

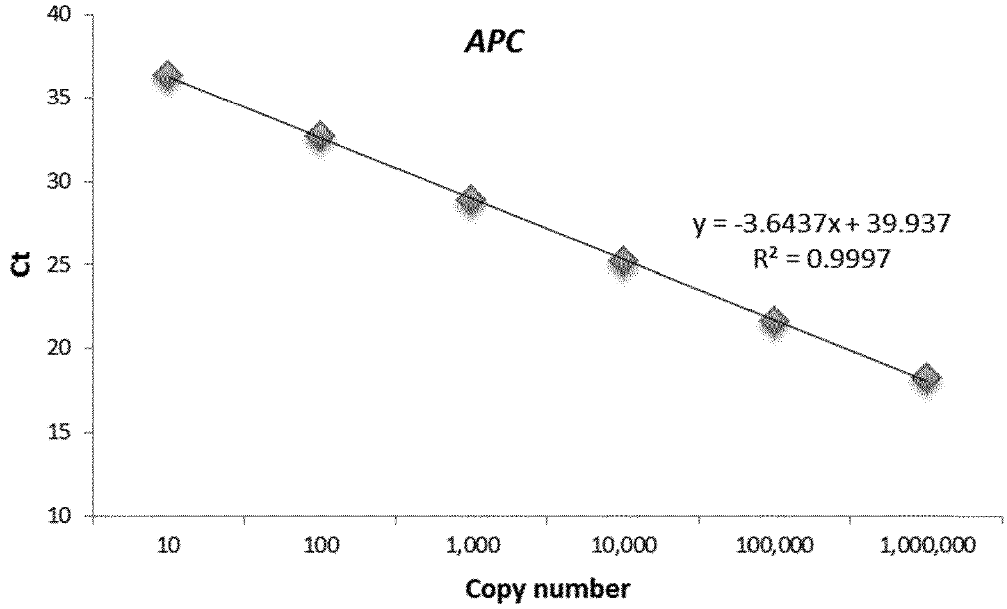


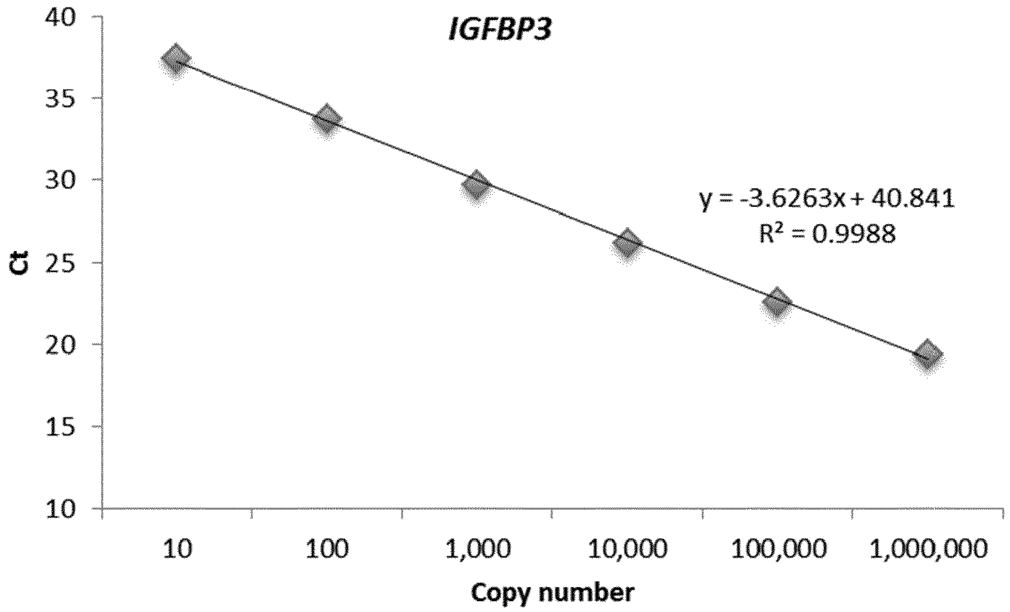


**Figure 5 (cont.)**

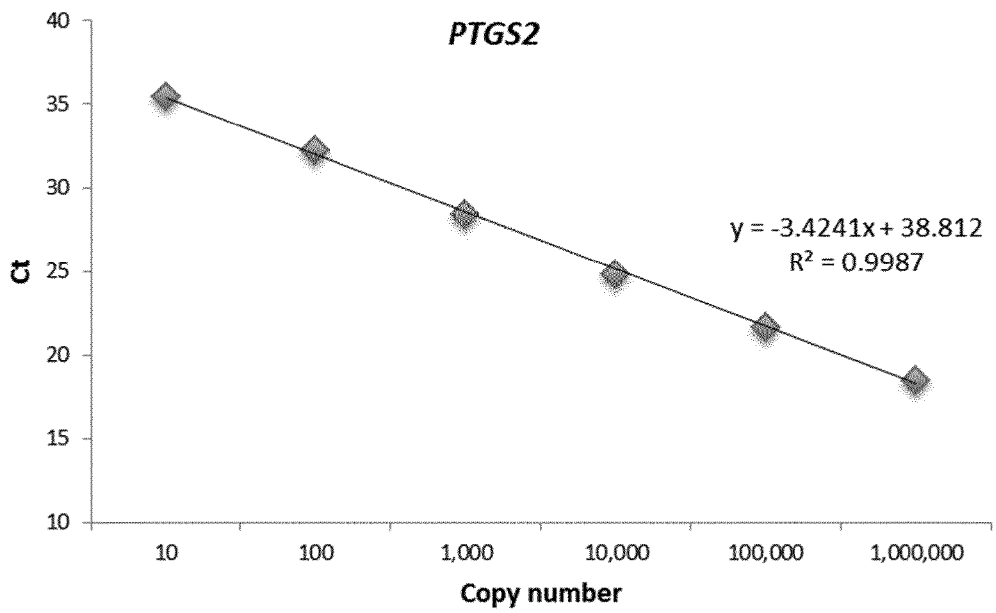


Figure 5 (cont.)

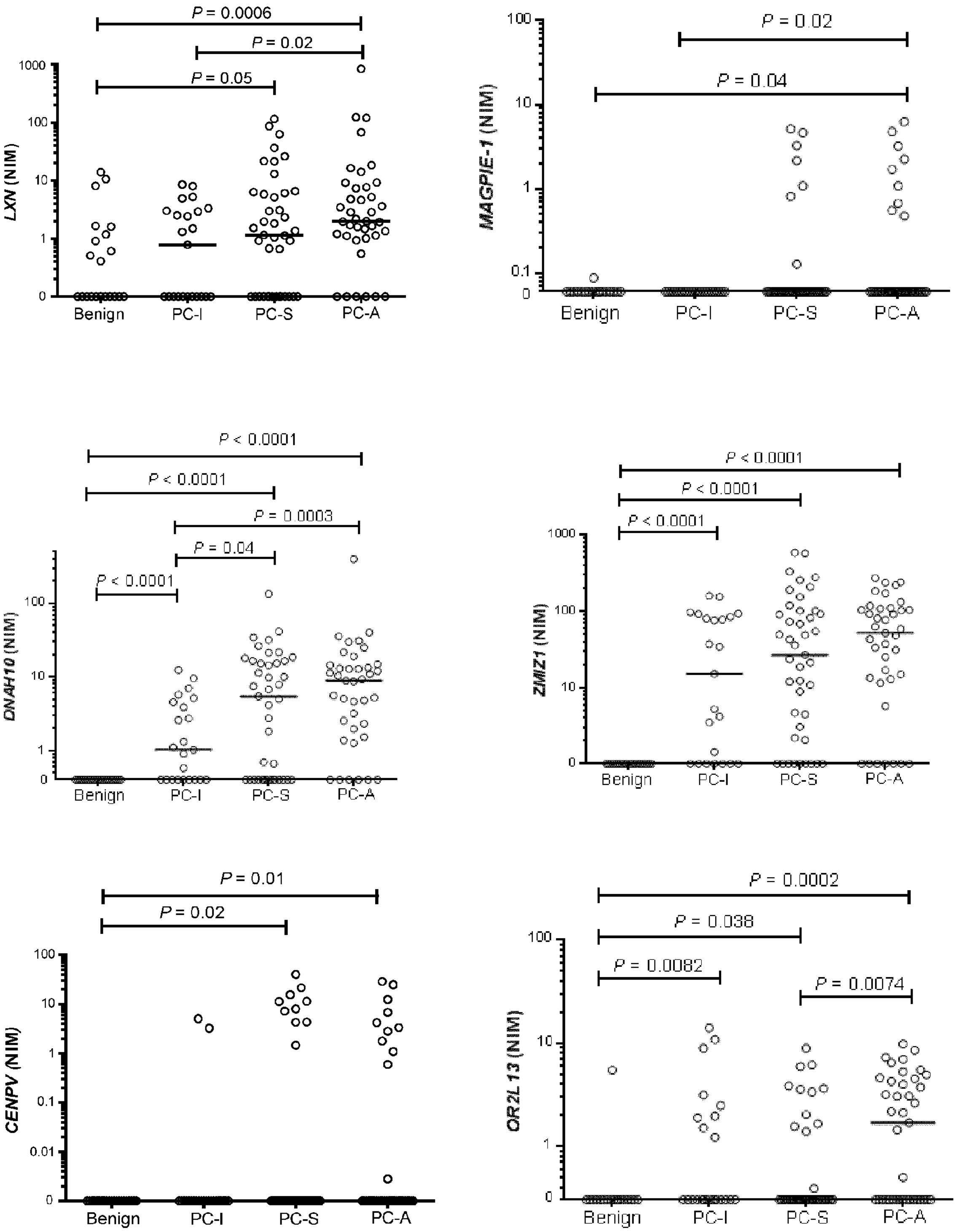
P = 0.0006
P = 0.02
P = 0.05
LXN (NIM)
1000
100
10
1
0
Benign
PC-I
PC-S
PC-A
P = 0.02
P = 0.04
MAGPIE-1 (NIM)
100
10
1
0.1
0
Benign
PC-I
PC-S
PC-A
P < 0.0001
P < 0.0001
P = 0.0003
P = 0.04
P < 0.0001
DNAH10 (NIM)
100
10
1
0
Benign
PC-I
PC-S
PC-A
P < 0.0001
P < 0.0001
P < 0.0001
ZMIZ1 (NIM)
1000
100
10
0
Benign
PC-I
PC-S
PC-A
P = 0.01
P = 0.02
CENPV (NIM)
100
10
1
0.1
0.01
0
Benign
PC-I
PC-S
PC-A
P = 0.0002
P = 0.038
P = 0.0082
P = 0.0074
OR2L13 (NIM)
100
10
1
0
Benign
PC-I
PC-S
PC-A

Figure 6

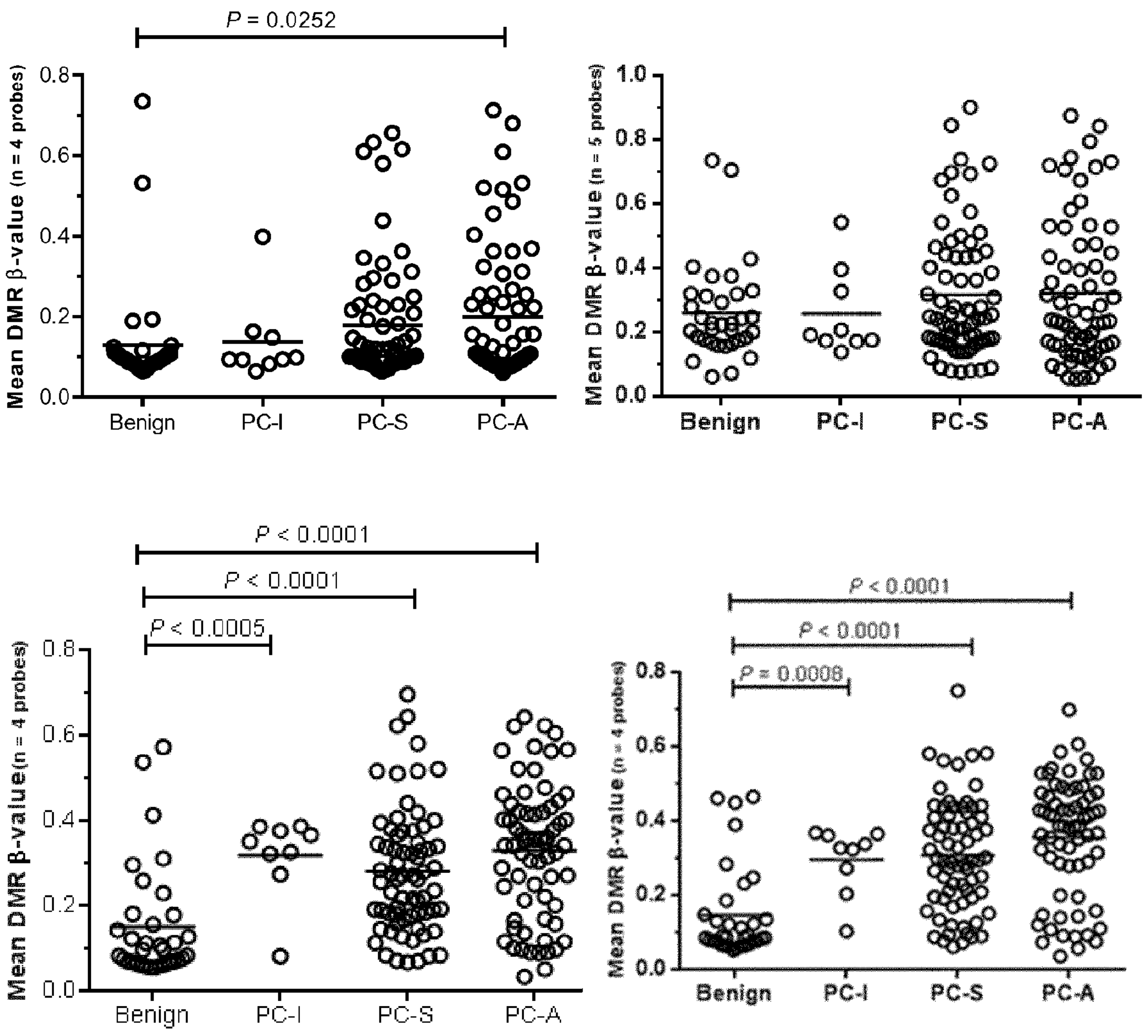


Figure 6 (cont.)

Europäisches Patentamt
European Patent Office
Office européen des brevets

# EUROPEAN SEARCH REPORT

Application Number
EP 14 19 9999

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 918 710 A1 (VERIDEX LLC [US]) 7 May 2008 (2008-05-07) * claims 1-7 * | 1-3,5-7 | INV. C12Q1/68 |
| X | WO 2013/041731 A1 (UNIV LEUVEN KATH [BE]; LITOVKIN KYRYLO [UA]; VAN EYNDE ALEYDE [BE]; BO) 28 March 2013 (2013-03-28) * claims 6,9,20 * | 1,2,5 | |
| X | EP 2 213 749 A1 (EPIGENOMICS AG [DE]) 4 August 2010 (2010-08-04) * claim 4; examples 1-3 * | 1 | |
| A | EP 1 780 292 A1 (VERIDEX LLC [US]) 2 May 2007 (2007-05-02) * the whole document * | 6 | |
| A | WO 2014/012176 A1 (DIAGNOCURE INC [CA]) 23 January 2014 (2014-01-23) * claim 12 * | 6 | TECHNICAL FIELDS SEARCHED (IPC) |
| A | WO 2007/022146 A2 (HARVARD COLLEGE [US]; MARSIT CARMEN J [US]; KARAGAS MARGARET R [US]; A) 22 February 2007 (2007-02-22) * page 19 * | 6 | C12Q |

The present search report has been drawn up for all claims

1

| Place of search | Date of completion of the search | Examiner |
|---|---|---|
| The Hague | 22 June 2015 | Gabriels, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number

EP 14 19 9999

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

## LACK OF UNITY OF INVENTION SHEET B

Application Number
EP 14 19 9999

The Search Division considers that the present European patent application does not comply with the requirements of unity of invention and relates to several inventions or groups of inventions, namely:

1. claims: 1-14

A method, assay and kit for determining the risk of aggressive prostate cancer in an individual diagnosed with prostate cancer, the method comprising a step of assaying a biological sample obtained from the individual for the presence of the methylated regulatory DNA sequence with SEQ ID No 1, or variants thereof, and correlating the presence or absence of the methylated regulatory DNA sequence with aggressive prostate cancer.

1.1. claims: 1-14

The same as above but limited to the next SEQ ID whereby invention 2 corresponds to SEQ ID NO:2, invention 3 corresponds to SEQ ID NO:3, ..., and invention 16 corresponds to SEQ ID NO:16.

---

Please note that all inventions mentioned under item 1, although not necessarily linked by a common inventive concept, could be searched without effort justifying an additional fee.

# ANNEX TO THE EUROPEAN SEARCH REPORT ON EUROPEAN PATENT APPLICATION NO. EP 14 19 9999

This annex lists the patent family members relating to the patent documents cited in the above-mentioned European search report. The members are as contained in the European Patent Office EDP file on
The European Patent Office is in no way liable for these particulars which are merely given for the purpose of information.

22-06-2015

| Patent document cited in search report | Publication date | Patent family member(s) | Publication date |
|---|---|---|---|
| EP 1918710 A1 | 07-05-2008 | CA 2610104 A1 | 30-04-2008 |
| | | CN 101220392 A | 16-07-2008 |
| | | EP 1918710 A1 | 07-05-2008 |
| | | JP 2008206511 A | 11-09-2008 |
| | | KR 20080039325 A | 07-05-2008 |
| WO 2013041731 A1 | 28-03-2013 | WO 2013041731 A1 | 28-03-2013 |
| | | WO 2013149904 A1 | 10-10-2013 |
| EP 2213749 A1 | 04-08-2010 | AT 438740 T | 15-08-2009 |
| | | AU 2005322435 A1 | 06-07-2006 |
| | | CA 2593546 A1 | 06-07-2006 |
| | | EP 1831399 A2 | 12-09-2007 |
| | | EP 2213749 A1 | 04-08-2010 |
| | | EP 2280084 A1 | 02-02-2011 |
| | | EP 2284279 A1 | 16-02-2011 |
| | | EP 2311984 A1 | 20-04-2011 |
| | | ES 2331146 T3 | 22-12-2009 |
| | | ES 2534139 T3 | 17-04-2015 |
| | | JP 2008522590 A | 03-07-2008 |
| | | JP 2012120538 A | 28-06-2012 |
| | | US 2009197250 A1 | 06-08-2009 |
| | | WO 2006071466 A2 | 06-07-2006 |
| EP 1780292 A1 | 02-05-2007 | AU 2006235762 A1 | 17-05-2007 |
| | | BR PI0604502 A | 28-08-2007 |
| | | CA 2566983 A1 | 30-04-2007 |
| | | CL 2006002950 A1 | 22-02-2008 |
| | | CN 1966727 A | 23-05-2007 |
| | | EP 1780292 A1 | 02-05-2007 |
| | | JP 2007125014 A | 24-05-2007 |
| | | KR 20070046764 A | 03-05-2007 |
| | | US 2007122818 A1 | 31-05-2007 |
| WO 2014012176 A1 | 23-01-2014 | CA 2879557 A1 | 23-01-2014 |
| | | CN 104603292 A | 06-05-2015 |
| | | EP 2875157 A1 | 27-05-2015 |
| | | WO 2014012176 A1 | 23-01-2014 |
| WO 2007022146 A2 | 22-02-2007 | CA 2619694 A1 | 22-02-2007 |
| | | US 2009155786 A1 | 18-06-2009 |
| | | WO 2007022146 A2 | 22-02-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5962233 A
- US 5538848 A
- US 5639606 A
- US 5643765 A
- US 5876978 A


### Non-patent literature cited in the description


- **TIBSHIRANI R.** Regression shrinkage and selection via the LASSO. *J. Royal. Statist. Soc B.,* 1996, vol. 58 (1), 267-288
- **BASTIAN PJ ; BOORJIAN SA ; BOSSI A et al.** High-risk prostate cancer: from definition to contemporary management. *European urology,* 2012, vol. 61 (6), 1096-106
- **EADS CA DK ; KAWAKAMI K ; SALTZ LB ; BLAKE C ; SHIBATA D ; DANENBERG PV ; LAIRD PW.** MethyLight: a high-throughput assay to measure DNA methylation. *Nucleic Acids Research,* 2000, vol. 28 (8), E32
- **PERRY AS ; LOFTUS B ; MOROOSE R et al.** In silico mining identifies IGFBP3 as a novel target of methylation in prostate cancer. *British journal of cancer,* 2007, vol. 96 (10), 1587-94
- **PERRY AS ; O'HURLEY G ; RAHEEM OA et al.** Gene expression and epigenetic discovery screen reveal methylation of SFRP2 in prostate cancer. *International journal of cancer Journal international du cancer,* 2013, vol. 132 (8), 1771-80
- **YEGNASUBRAMANIAN S ; KOWALSKI J ; GONZALGO ML et al.** Hypermethylation of CpG islands in primary and metastatic human prostate cancer. *Cancer Res,* 2004, vol. 64 (6), 1975-86
- **HASTIE TAF.** The elements of statistical learning. Springer-Verlag, 2009